(19) Europäisches Patentamt
European Patent Office
Office européen des brevets

(11) EP 1 881 065 A1

(12) **EUROPEAN PATENT APPLICATION**

(43) Date of publication:
**23.01.2008 Bulletin 2008/04**

(51) Int Cl.:
***C12N 9/00*** *(2006.01)*

(21) Application number: **07105231.0**

(22) Date of filing: **26.03.2002**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **19.04.2001 US 284987 P**
**27.03.2001 US 279188 P**
**04.06.2001 US 295726 P**

(62) Document number(s) of the earlier application(s) in accordance with Art. 76 EPC:
**02726212.0 / 1 373 485**

(71) Applicants:
- **Novartis AG**
**4056 Basel (CH)**
Designated Contracting States:
**BE BG CH CY CZ DE DK EE ES FI FR GB GR HU IE IS IT LI LT LU LV MC MT NL PL PT RO SE SI SK TR**
- **Novartis Pharma GmbH**
**1230 Vienna (AT)**
Designated Contracting States:
**AT**

(72) Inventors:
- **De Martin, Rainer**
**1120 Wien (AT)**
- **Schmid, Johannes**
**2340 Mödling (AT)**
- **Hofer-Warbinek, Renate**
**1238 Wien (AT)**
- **Hofer, Erhard**
**1238 Wien (AT)**
- **Kalthoff, Frank Stephan**
**2353 Guntramsdof (AT)**
- **Lipp, Hans-Joachim**
**2500 Baden (AT)**
- **Mechtcheriakova, Diana**
**1130 Wien (AT)**
- **Sobanov, Yuri**
**1050 Wien (AT)**

(74) Representative: **Schaller, Hans**
**Novartis AG**
**Corporate Intellectual Property**
**4002 Basel (CH)**

Remarks:
This application was filed on 29 - 03 - 2007 as a divisional application to the application mentioned under INID code 62.

(54) **DINO polypeptide**

(57) An isolated IKK2-55 gene and polypeptide; DINO gene and polypeptide; LLR-J24-stalk nucleotide and polypeptide, a polynucleotide encoding the transmembrane protein LLR-J24 and the transmembrane protein LLR-J24; a polynucleotide encoding the extracellular domain of LLR-J24 and a polypeptide which is the extracellular domain of LLR-J24; their use in a method of identifying an agonist or antagonist of such polypeptide/protein; and an antagonist or an agonist of such polypeptide/protein and its use as a pharmaceutical.

EP 1 881 065 A1

## Description

The invention relates to polynucleotide encoding a polypeptide (protein) isolated from PHA-activated lymphocytes, endothelial cells, or dendritic cells (DCs). The invention also relates to inhibiting or activating the action of such polynucleotide and/or polypeptide (protein).

Endothelial cells (EC) make up the inner surface of large and small blood vessel and mediate the transmigration of cells of the immune system into the underlying tissue. Non-stimulated EC represent an anticoagulant surface that ensures unobstructed blood flow. Upon stimulation with inflammatory mediators such as TNF$\alpha$, IL-1 or lipopolysaccharide (LPS), EC express a variety of molecules including cell adhesion molecules (including e.g. E-selectin, ICAM-1 and VCAM-1), cytokines (IL-1, IL-6, IL-8), components of the coagulation system (tissue factor, PAI-1) and others (iNOS). These expressed molecules mediate chemotaxis, adhesion and transmigration of immune cells, as well as several other functions (de Martin et al. Arterioscler. Thromb. Vasc. Biol. 20 [2000] e83-e88.). The inflammatory response is therefore a common underlying mechanism for a variety of diseases including e.g. inflammation, arteriosclerosis, restenosis, reperfusion injury, inflammatory bowel disease, psoriasis and atopic dermatitis.

The vast majority of the expressed molecules mentioned above is up-regulated in an inducible and transient way that includes the transcription factor NF-kB. Inhibition of NF-kB by pharmacological or genetic inhibitors has been shown to block the inflammatory and other aspects of EC activation (Wrighton et al. J. Exp. Med. 183 [1996] 1013-1022; Oitzinger et al. Blood 97 [2001] 1611-1617). Inhibitors of NF-kB have therefore wide therapeutic potential.

The current understanding of the regulatory steps that lead to activation of NF-kB includes the following: upon stimulation, NF-kB (e.g. the p65/p50 heterodimer) is released from an inactive cytoplasmic complex that contains the inhibitory subunit IkBa, -b or -e. IkB subunits, when bound to NF-kB, mask the NF-kB nuclear localization signal and thus prevent its nuclear translocation. Liberation of NF-kB is initiated by IkB phosphorylation on two serine residues, Ser 32 and Ser 36 in IkBa and corresponding sites in IkBb followed by recognition by the b-TrCP-like component of an E3 ubiquitin ligase complex (Skp1/Cul1/ROC1/F-box protein FWD1), ubiquitinylation and degradation via the 26S proteasome.

A major breakthrough in the field has been the identification of two kinases (IKK1/IKKa and IKK2/IKKb) that specifically phosphorylate IkBa. These kinases form homo- and heterodimers through their leucine zipper domains and are bound to a third protein (NEMO/IKKg). This IkB kinase complex (IKC), consisting of IKK1, IKK2 and NEMO, is included within a high molecular weight (500-700 kD) complex termed signalosome, which may contain other proteins such as the catalytic subunit of PKA (csPKA) and phospho-tyrosine containing proteins. Genetic evidence using knockout mice has revealed a prominent role for IKK2 in inflammatory signalling, whereas IKK1 appears to be involved in developmental processes. In addition to the IKKs, pp90rsk and DNA-PK have been demonstrated to be capable of directly phosphorylating and activating IkB. In addition, two novel IKKs have been described, one that is inducible by LPS on the mRNA level in macrophages (IKK-i) and one (TBK1) that forms a complex with TRAF2 and TANK and thus represents an alternative signalling pathway for TNFa leading to NF-kB activation. Several groups have described modulators that influence IKK activity either positively or negatively (e.g. TAK1; Hofer-Warbinek et al. J. Biol. Chem. 275 [2000] 22064-22068), indicating additional important levels of regulation of NF-kB activity.

In different cell types, multiple upstream activators of the IKC have been described. These include the MAP3K type kinases NIK (NF-kB inducing kinase) and MEKK1 (MAPK/ERK kinase kinase 1), the TGFb-inducible kinase TAK1 as well as Akt and the PKC isoforms zeta and theta, reflecting both heterogeneity but also a certain degree of specificity of the NF-kB signalling pathway in regard to different cell types and stimuli. These upstream activators couple to different components of the receptor-specific cytoplasmatic signalling complexes that are recruited to the receptors upon stimulation, e.g. TRAF1, TRAF2 and TRADD for TNF-Rs, and TRAF6 for IL-1R and Toll family receptors. Thus, IKK2 appears to be of central importance in NF-kB signalling.

It has now been found that from cDNA libraries of isolated PHA-activated lymphocytes **an IKK2-55 gene** may be isolated which has the nucleotide sequence of e.g. **SEQ ID NO:1** and which encodes a **polypeptide** which has the amino acid sequence set forth in **SEQ ID NO:2** or at least 80 % identity thereto.

Endothelial cells (EC) are localized to the vasculature. They are connected by junctional complexes and form the endothelium, which lines the entire vascular system. They control substance flow between the circulation and the specific organ and are the primary sites of inflammatory responses. Moreover, they are the sites where interactions between circulating cells of the body's own immune defense system and altered cells of the specific organ occur. Exchange of substances, peptides and immune cells between the blood stream and various tissues of the body are controlled by the EC barrier. The selectivity of this EC barrier is critical for the formation of several so-called "blood-tissue" barriers which are indispensable for maintaining the integrity of the respective organ. In response to different pathological stimuli, EC may adjust their constitutive functions, resulting in several types of reversible changes of their functional state, referred to as endothelial dysfunction. EC have to respond in a balanced manner to a multitude of physiological signals which are essential in maintenance of a proper blood flow, providing oxygenation and nutrition of all tissues, and fighting microbial, viral and parasitic infections. In addition, EC are also involved in wound repair by induction of growth and regression of new blood vessels, cancer progression, and atherosclerotic lesion development (see e.g. Cines et al. Blood 91 [1998] 3527-3561).

Capillary EC may be isolated from human tissue by immunomagnetic beads using specific monoclonal antibodies or Ulex europaeus lectin type 1 coated to magnetic beads (see e.g. Manconi et al. Meth. Cell Science [2000] 22 89-99). cDNA libraries of EC may be generated and gene expression patterns of EC may be obtained by various hybridization techniques such as oligonucleotide fingerprinting, subtractive hybridization or RNA profiling, and sequencing in conventional manner.

It has now also been found that from cDNA libraries of isolated ECs **a DINO gene** may be isolated which has the nucleotide sequence of e.g. **SEQ ID NO:3** and which encodes a DINO **polypeptide** which has the amino acid sequence set forth in **SEQ ID NO:4** or at least 80 % identity thereto.

Dendritic cells (DC) are professional antigen presenting leukocytes which play a central role in the induction of primary immune responses and tolerance. In the immature state DCs may reside in different tissues of the body being prepared to capture antigen from invading pathogens. Following antigen capture DC mature into antigen-presenting cells and migrate into lymphoid organs to activate T cells (Banchereau, M. and R.M. Steinman Nature 329 [1998] 245-252). For example, Langerhans cells (LC), residing in the skin, have been shown to present a variety of antigens that may be generated in or penetrate into skin. In contact hypersensitivity, topical application of a reactive hapten may activate LC to migrate out of the epidermis into draining lymph nodes, where LC may present antigen to selected T cells. During the contact between LCs and T-cells, LCs may provide signals to the T-cells that induce their proliferation and differentiation into effector cells. Depending on the type of T-cells and the kind of interacting molecules involved cytotoxic, regulatory and helper T-cells may be formed. DC's have also been shown to engulf all kinds of apoptotic cells and may therefore play a critical role in the maintenance of tolerance to self-antigens (Steinman R.M. and K. Inaba J. Leukoc. Biol. 66 [1999] 205-208). Diseases in which DC's, as the principal regulators of immune responses, play a causal or contributory role may be targets for DC-specific pharmaceutical or iatrigenic intervention such as in chronic inflammatory diseases, autoimmune diseases, or transplant rejection crisis, and including inflammatory skin diseases such as contact hypersensitivity or atopic dermatitis, and in diseases or syndromes in which a significant pathological component is immune suppression, as in AIDS and cancer.

DCs may be isolated from peripheral blood by negative selection, i.e. separation from monocytes (CD14+), T-cells (CD3+), B-cells (CD19+) and NK (CD16+)-cells by capturing on specific mAB coated magnetic beads or panning, e.g. according to a conventional method. cDNA libraries of DC's may be generated and gene expression patterns of DC may be obtained by various hybridisation techniques such as oligonucleotide fingerprinting, subtractive hybridisation or RNA profiling, and sequencing, e.g. according to a conventional method. Transmembrane receptors of DCs are of importance, since on the one hand they are involved in the uptake of parasites and apoptotic cells leading to antigen presentation by the DCs. On the other hand these receptors interact with soluble ligands and surface bound ligands on T cells and other immune cells. These interactions are involved in regulating DC function as well as the functions of the interacting cells such as T cells. These regulatory events, depending on the differentiation state of the cell and the interacting ligands, include activation and inhibition of DCs, T cells and other immune cells.

Receptors with type II transmembrane orientation and a C-type lectin-like domain in their extracellular part have been found to be frequently expressed on various hemopoietic cells, and some have been shown to play important roles in the innate immune response (Weis W.I., Taylor M. E. and Drickamer K. Immunol. Rev. 163 [1998] 19-34). Aside proteins such as CD69 and AICL (Santis A.G. et al. Eur. J. Immunol. 24 [1994] 1692-7; Hamann et al., Immunogenetics 45 [1997] 295-300), which are widely expressed in cells of the hemopoietic lineage, some are more restricted in cell type expression, such as the lectin-like NK cell receptors which have recently been intensively investigated. The lectin-like NKR-P1 and Ly49 genes (Yokoyama W. M. et al. J. Immunol. 147 [1991] 3229-3236), initially detected in rodent NK cells, and the CD94 and NKG2 genes (Chang C. et al. Eur. J. Immunol. 25 [1995] 2433-2437; Houchins J. P. et al. J. Exp. Med. 173 [1991] 1017-1020; Hofer E. et al. Immunol. Today 13 [1992] 429-430), described first from human NK cells, occur on syntenic regions of mouse chromosome 6 (Brown M.G. et al. Genomics 42 [1997] 16-25), rat chromosome 4 and on human chromosome 12p12.3-p13.2 (Sobanov Y. et al. Immunogenetics 49 [1999] 99-105). These regions have therefore been termed NK gene complex. Consistent with the idea of a chromosomal area containing several genes important for natural immune cell function, loci mediating resistance to virus infection or mediating lysis of allogeneic lymphocytes have been mapped to this region in rodents.

It is now well established that the lectin-like NK receptors possess important roles for monitoring proper MHC class I expression (Lopez-Botet M. et al. Semin. Immunol. 12 [2000] 109-119; Hoglund P. Immunol. Rev. 155 [1997] 11-28). In human NK cells the CD94 chain forms heterodimeric receptors with different NKG2 isoforms, which can bind the non-classical MHC class Ib molecule HLA-E. CD94/NKG2A functions as an inhibitory receptor, the CD94/NKG2C receptor can activate NK cells. HLA-E predominatly presents peptides from the leader sequences of other class Ia molecules and is only transported to the surface when loaded with the appropriate peptides. Thus recognition of HLA-E by the CD94/NKG2A receptor is a sensitive mechanism to monitor proper biosynthesis of MHC class I molecules, which can be down-modulated by many viruses (Alcami A. and U.H. Koszinowski Immunol. Today 21 [2000] 447-55).

The function of the activating CD94/NKG2C receptor in this context is still not understood. The activating NKG2D receptor, only distantly related to the other NKG2 molecules, forms homodimers and recognizes the class I-like molecule MICA, which is upregulated by stress, e.g. viral infection, and on some tumor cells (Bauer S. et al., Science 285 [1999] 727-729). The corresponding CD94 and NKG2 receptors in mice appear to have comparable functions.

The genomic structure and transcription of the human NK receptor genes have been previously investigated (Glienke J. et al. Immunogenetics 48 [1998] 163-173) as well as the function of their protein products (Duchler M. et al. Eur. J. Immunol. 25 [1995] 2923-31). In an attempt to find genes related in evolution and function to the NKG2 and CD94 receptor genes, these studies have been extended to cover areas of the NK gene complex flanking these genes on both sides for about 1 Mb. Whereas it has not been possible to detect any further human Ly49 genes on the centromeric side, besides the Ly49L pseudogene (Bull C. et al. Genes and Immunity 1 [2000] 280-287), a **novel cluster of 4 related lectin-like genes** telomeric of CD94 has now been found. This cluster contains the LOX-1 (Sawamura T. et al. Nature 386 [1997] 73-77), the novel human lectin-like receptor (LLR-J24) gene and the recently described Clec-1 and Clec-2 genes (Colonna M., Samaridis J., Angman L. Eur. J. Immunol. 30 [2000] 697-704). Their chromosomal localization, expression pattern and their similarity to the NK receptor genes suggests important innate immune functions in DCs, monocytes and endothelial cells.

DCs are antigen-presenting cell populations in lymphoid and non-lymphoid organs (Hart D.N. Blood 90 [1997] 3245-87). They play important roles in the initiation of antigen-specific T cell proliferation. Immature DCs occur in peripheral non-lymphoid organs, where their principal function is to filter the surrounding tissues for foreign antigens and pathogens. These immature DCs are highly specialized in antigen uptake and processing and stay for long time periods in non-lymphoid tissues. Following antigen uptake these DCs are capable to migrate to T cell-rich areas of secondary lymphoid organs such as lymph nodes or spleen. This is associated with profound phenotypic and functional changes known as DC maturation. In this process DCs upregulate MHC class I and II molecules as well as T cell costimulatory molecules such as CD80 and CD86. Mature DCs are potent stimulators of helper and cytotoxic T cells. Understanding the molecular signals which guide the differentiation of DCs, which in turn program differentiation of lymphocytes is of key importance to be able to manipulate the immune response more efficiently with regard to vaccination strategies or to switch off damaging immune responses. Based on the present finding of the specific expression of LLR-J24 in DCs and its down-modulation during the maturation process it is conceivable that this receptor plays a role during DC maturation or T cell stimulation.

A **gene LLR-J24** has now been found, isolated from and selectively expressed in monocyte-derived and cord blood progenitor cell-derived DCs (MoDC and CBDC). The **LLR-J24-1** gene of nucleotide **SEQ ID NO:5** and its splice variant **LLR-J24-2** of nucleotide **SEQ ID NO:7** are related to but not identical to the NKG2 and CD94 NK receptor chains (Houchins J.P. et al., J. Exp. Med. 173 [1991] 1017-1020; Chang C. et al., Eur. J. Immunol. 25 [1995] 2433-7; GenBank accession No.: NKG2A [X54867], NKG2C [X54869], NKG2E [L14542], NKG2H [AF078550], NKG2D [X54870]), the oxLDL receptor LOX-1 (Sawamura T. et al. Nature 386 [1997] 73-77; GenBank accession No. AF035776) and two other receptor genes recently found to be preferentially expressed in DCs, CLEC-1 and CLEC-2 (Colonna M. et al. Eur. J. Immunol. 30 [2000] 697-704; GenBank accession No. CLEC-1 [AF200949], CLEC-2 [AF124841]). A somewhat more distant relationship exists to other members of the so-called NK receptor gene complex on human chromosome 12 (Sobanov Y. et al. Immunogenetics 49 [1999] 99-105). The LLR-J24-1 gene may occur in isolated DC-s as the splice variant **LLR-J24-2** which consists of the nucleotide sequence of **SEQ ID NO:5** and an additional exon encoding a **stalk peptide** inserted between nucleotides 357 to 358. Thus the splice variant **SEQ ID NO:5** is identical to **SEQ ID NO:7** but misses nucleotides 358 to 495 which are spliced out.

The LLR-J24 gene encodes a novel member of the superfamily of type II transmembrane receptors with a C-type lectin-like domain (CTLD) in their extracellular part (Weis W. I. et al. Immunol. Rev. 163 [1998] 19-34). Its closest relatives are the mouse DECTIN-1 (59 % identity, Ariizumi et al. J. Biol. Chem. 275 [2000] 20157-20167, accession No. AF262985), the human LOX-1 (44 % similarity in CTLD, Sawamura T. Nature 386 [1997] 73-77; accession No. AF035776), CLEC-1 (37 % similarity in CTLD, Colonna M. et al. Eur. J. Immunol. 30 [2000] 697-704, accession No. AF200949), CLEC-2 (37 % similarity in CTLD, Colonna M. et al. Eur. J. Immunol. 30 [2000] 697-704, accession No. AF124841), NKG2-D (33 % similarity in CTLD, Houchins J.P. et al. J. Exp. Med. 173 [1991] 1017-1020, accession No. X54870), NKG2-A (30 % similarity in CTLD, accession No. X54867), NKG2-C (30 % similarity in CTLD, accession No. X54869) and CD94 receptor (31 % similarity in CTLD, Chang C. et al. Eur. J. Immunol. 25 [1995] 2433-7) chains. The LLR-J24 gene is located on chromosome 12 in the NK receptor gene complex, which also contains the above-mentioned homologues and additional somewhat more distantly related genes. The LLR-J24-1 gene of **SEQ ID NO:5** and **NO:7** encodes a typical CTLD and an ITAM-like sequence in its cytoplasmic tail. The sequence of the CTLD is clearly homologous to all the CTLDs of the other members of the superfamily, the cytoplasmic tail is only distantly related or unrelated to the other family members, but clearly homologous to the mouse DECTIN-1 sequence. The LLR-J24-2 gene of **SEQ ID NO: 7** is identical to **SEQ ID NO:5,** but contains an additional exon encoding a stalk peptide. The corresponding protein with the stalk peptide is shown in **SEQ ID NO:8.** The stalk exon nucleotide sequence is depicted as **SEQ ID NO:9,** and the corresponding stalk peptide as **SEQ ID NO:10.**

It was now found that the stalk peptide is necessary for the expression of the receptor on the cell surface, the receptor without the stalk peptide is expressed intracellularly. The CTLD binds surface peptide structures on other immune cells such as T cells. This may provide a modulating, e.g. costimulatory signal to T cell activation. The CTLD binds also surface structures on other immune cells such as T cells. The receptor is downregulated during DC maturation. This may relieve also an inhibitory signal to T cell activation. The cytoplasmic domain of the receptor regulates DC maturation upon binding of the CTLD to the ligand. The CTLD binds e.g. lipoprotein structures such as membrane structures of parasites, oxLDL and apoptotic cell membranes. Cells and compounds containing these structures are internalized leading to efficient antigen-presentation. The receptor is downregulated during DC maturation when the phagocytotic activity of DC diminishes. The cytoplasmic domain of the receptor mediates internalization and activation of DC maturation upon binding of the CTLD to the ligand.

The intracellular version of the receptor without the stalk peptide is involved in monitoring aberrant intracellular structures and parasites and has thus another function than the version comprising the stalk peptide of **SEQ ID NO:10.**

The invention thus provides an isolated

- **IKK2-55 gene** comprising a nucleotide sequence encoding an IKK2-55 polypeptide of **SEQ ID NO:2;**
- **DINO gene** comprising a nucleotide sequence encoding a DINO polypeptide of **SEQ ID NO:4;** or
- **LLR-J24-stalk nucleotide** comprising a nucleotide sequence encoding a LLR-J24-stalk peptide of **SEQ ID NO:10.**

The invention further provides an isolated

- **IKK2-55 gene** comprising the nucleotide sequence of e.g. **SEQ ID NO:1,** encoding an IKK2-55 polypeptide of SEQ ID NO:2;
- **DINO gene** comprising the nucleotide sequence of e.g. **SEQ ID NO:3,** encoding a DINO polypeptide of SEQ ID NO:4; or
- **LLR-J24-stalk nucleotide** comprising the nucleotide sequence of e.g. **SEQ ID NO:9,** encoding a stalk peptide of SEQ ID NO:10.

The invention further provides an isolated

- **IKK2-55 polypeptide of SEQ ID NO:2;**
- **DINO polypeptide of SEQ ID NO:4; or**
- **LLR-J24 stalk peptide of SEQ ID NO:10.**

The IKK2-55 gene, the DINO gene, the LLR-J24-stalk gene, an isolated polynucleotide encoding the transmembrane protein LLR-J24 and an isolated polynucleotide encoding the extracellular domain of LLR-J24 are designated herein as **"Gene(s) according to (of) the invention".**

The IKK2-55 polypeptide, the DINO polypeptide, the LLR-J24-stalk peptide, an isolated polypeptide which is the extracellular domain of LLR-J24 and an isolated polypeptide which is the transmembrane protein LLR-J24 are designated herein as **"Polypeptide(s) according to (of) the invention".**

A polypeptide of the invention includes a polypeptide of the amino acid sequences of **SEQ ID NO:2, SEQ ID NO:4 and SEQ ID NO:10,** and includes e.g. an amino acid sequence which has at least 80 % identity with the corresponding amino acid sequences of **SEQ ID NO:2, SEQ ID NO:4** or **SEQ ID NO:10,** respectively, and the same biological activity as a corresponding polypeptide of the invention. A polypeptide of the invention includes a polypeptide which is encoded by a corresponding gene of the invention, including a polynucleotide that hybridizes to the nucleotide sequence of a gene of the invention; e.g. including a sequence, which as a result of the redundancy (degeneracy) of the genetic code, also encodes a corresponding polypeptide of the invention; or e.g. an allelic variant and/or complement of a gene of the invention.

A polypeptide of the invention may be in the form of the "mature" polypeptide, e.g. protein, or may be part of a larger polypeptide, e.g. protein, e.g. of a fusion protein; it may be advantageous to include an additional amino acid sequence which contains e.g. secretory or leader sequences, pro-sequences, sequences which aid in purification, such as multiple histidine residues, or an additional sequence for stability during recombinant production into a polypeptide of the invention.

A polypeptide of the invention also includes a polypeptide fragment of a polypeptide of the invention. Such polypeptide fragment is meant to be a polypeptide having an amino acid sequence that entirely is the same in part, but not in all, of the amino acid sequence of a polypeptide of the invention. Such polypeptide fragment may be "free-standing," or may be part of a larger polypeptide of which such polypeptide fragment forms a part or region, most preferably as a single continuous region. Preferably such polypeptide fragment retains the biological activity of the corresponding polypeptide of the invention.

Variants of defined polypeptide (fragment) sequences of the invention also form part of the present invention. Preferred variants are those that vary from the referents by conservative amino acid substitutions, e.g. those that substitute a residue with another of like characteristics. Typically such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5 to 10, 1 to 5, or 1 or 2 amino acids are substituted, deleted or added, in any combination.

A polypeptide of the invention, or fragment thereof, includes

- isolated naturally occurring polypeptides
- recombinantly produced polypeptides
- synthetically produced polypeptides and
- a combination of such polypeptides

of the invention, and fragments thereof.

A polypeptide of the invention or fragment thereof may be produced as appropriate, e.g. in conventional manner. "Isolated", if not otherwise specified herein includes the meaning "separated from the coexisting material", e.g. "altered by the hand of man", from the natural state.

A gene of the invention includes a corresponding nucleotide sequence of **SEQ ID NO:1, SEQ ID NO:3,** and **SEQ ID NO:9,** respectively, and allelic variants therof, and its complements, and splice variants therof; including e.g. a polynucleotide that hybridizes to a nucleotide sequence of a gene of the invention, e.g. under stringent conditions, e.g. each nucleotide sequence of a gene of the invention includes a sequence which is different, e.g. as a result of the redundancy (degeneracy) of the genetic code, from the sequence of a gene of the invention, but also encodes a corresponding polypeptide of the invention, or encodes a polypeptide of the invention of an amino acid sequence which has at least 80% identity with the amino acid sequence of **SEQ** ID **NO:2, SEQ ID NO:4,** or **SEQ ID NO:10,** respectively, and having the same biological activity as a polypeptide of the present invention. "Stringent conditions" includes that hybridization will occur only if there is at least 80 %, e.g. 90 %, such as 95 %, 97 % or 99 % identity between the nucleotide sequence of a gene of the invention and the corresponding polynucleotide that hybridizes therewith.

A gene of the invention encodes a corresponding polypeptide, or a part of a corresponding polypeptide (fragment), of the invention or encodes a polypeptide or a part of a polypeptide of an amino acid sequence which has at least 80 % identity with a correspondingly encoded polypeptide of the invention, e.g. over the entire lenghth of said corresponding amino acid sequence; e.g. 80 % to 100 %, such as 90 %, e.g. 95 %, e.g. 97 %, e.g. 99 % or 100 % identity; said identity being calculated by

$$n_a = x_a - (x_a \,.\, y)$$

wherein $n_a$ is the number of amino acid alterations, $x_a$ is the total number of amino acids in said corresponding amino acid sequence, and y is percent identity divided by 100; including e.g. a polypeptide encoded by an allelic variant of said gene, or an isoform of the corresponding amino acid sequence generated by alternative splicing of transcripts from the corresponding gene. "Identity" is a measure of the identiiy of nucleotide sequences or amino acid sequences and may be calculated by conventional techniques, using e.g. commercially available computer programs.

"Polypeptide ", if not otherwise specified herein, includes any peptide or protein comprising two or more amino acids joined to each other by peptide bonds. "Polynucleotide", if not otherwise specified herein, includes any polyribonucleotide or polydeoxyribonucleotide, which may be unmodified RNA or DNA, or modified RNA or DNA, including without limitation single and double stranded RNA, and RNA that is a mixture of single- and double-stranded regions; and RNA hybrid molecules.

A gene of the invention encoding a corresponding polypeptide of the invention may be obtained using standard cloning and screening methods, from a cDNA library derived from mRNA in PHA-activated lymphocytes or endothelial cells, or DCs, respectively, e.g. by reverse transcription PCR using mRNA of DCs, e.g. using the expressed sequence tag (EST) analysis (Adams M.D. et al. Science 252 [1991] 1651-1656; Adams M.D. et al. Nature 355 [1992] 632-634; Adams M.D. et al. Nature 377 Suppl.3 [1995] 174). A gene acording to the invention may also be obtained from natural sources, such as genomic DNA libraries, or may be synthesized according to a conventional method.

A gene of the invention is useful for the recombinant production of a corresponding polypeptide (fragment) of the invention and, if used for such recombinant production, the gene sequence may include the coding sequence for the mature polypeptide (fragment) by itself; or the coding sequence for the mature polypeptide (fragment) in reading frame with other coding sequences, such as those encoding a leader or secretory sequence, a pre- or pro- or prepro-protein sequence, or other fusion peptide portions. For example, a marker sequence can be encoded which facilitates purification of a fused polypeptide. The marker sequence may be an appropriate conventional marker sequence, e.g. a FLAG-tag (Sigma) or a hexahistidine peptide, as provided in the pQE vector (Qiagen, Inc.) and described in Gentz et al. Proc. Natl Acad. Sci. USA 86 [1989] 821-824, or an HA tag. Any gene of the invention may also contain non-coding 5' and 3' sequences, such as transcribed, non-translated sequences, splicing and polyadenylation signals, ribosome binding sites and sequences that stabilize mRNA.

A nucleotide sequence which is identical or sufficiently identical to a nucleotide sequence of a gene of the invention, e.g. including a fragment thereof or a splice variant therof, may be used as an hybridization probe for cDNA and genomic DNA to isolate full-length cDNAs and genomic clones encoding a corresponding polypeptide (fragment) of the invention; and e.g. to isolate cDNA and genomic clones of other genes (including genes encoding homologs and orthologs from species other than human) that have a high sequence similarity to a gene of the invention.

Hybridization may e.g. be carried out according to a conventional method. Typically a sequence similar to a gene sequence is 80 % identical, preferably 90 % identical, more preferably 95 % identical to that of a the gene (fragment) of the invention. A hybridization probe may e.g. comprise at least 15 nucleotides, e.g. at least 30 nucleotides, such as at least 50 nucleotides, preferably between about 30 and about 50 nucleotides; or the cDNA sequence corresponding to the coding region of the entire cDNA sequence or any parts thereof. To obtain a polynucleotide encoding a polypeptide of the invention, including homologs and orthologs from species other than human, any appropriate hybridization technique may be used, e.g. comprising the steps of screening an appropriate library under stringent hybridization conditions with a labeled probe having the corresponding polynucletoide sequence or that of a splice variant thereof or a fragment thereof, and isolating full-length cDNA and genomic clones containing said polynucleotide sequence.

Hybridization techniques, e.g. strigent, are well known. Stringent hybridization conditions are e.g. as defined above, or alternatively, conditions under overnight incubation at around 40°C in an appropriate solution, e.g. a solution comprising formamide, SSC, sodium phosphate, Denhardt's solution, dextran and salmon sperm DNA, e.g. comprising 50 % formamide, 5 x SSC (150 mM NaCl, 15 mM trisodium citrate), 50 mM sodium phosphate (pH 7.6), 5 x Denhardt's solution, 10 % dextran sulfate, and 20 $\mu$g/ml denatured, sheared salmon sperm DNA, followed by washing the filters in 0.1 x SSC at about 65°C.

In a further aspect the invention provides a **vector** comprising a gene, e.g. a polynucleotide, of the invention.

Such vector may be produced as appropriate, e.g. in conventional manner. An appropriate vector may be provided according to a conventional method. A vector comprising a gene of the present invention may be useful to obtain an expression system which is able to produce a polypeptide encoded by a gene of the invention recombinantly, e.g. in a host cell, such as in a compatible host cell. For recombinant production of a polypeptide of the present invention a host cell may e.g. be genetically engineered, e.g. by use of a vector comprising a gene of the invention, to incorporate into the host cell an expression system, or a part therof, for expressing a polypeptide (fragment) of the invention. Cell-free translation systems may also be used to produce a gene of the invention, e.g. using RNAs derived from a DNA construct of the present invention, e.g. in conventional manner.

In a further aspect the invention provides an **expression system** comprising a DNA or RNA molecule, e.g. isolated from the natural environment, e.g. comprising a pre-isolated gene of the invention, wherein said expression system or part thereof is capable of producing a polypeptide of the invention when said expression system or part thereof is present in a compatible host cell.

In a further aspect the invention provides:

- an isolated **host cell** comprising an expression system of the invention;
- **a process for producing a polypeptide** of the invention comprising culturing an isolated host cell comprising an expression system of the invention under conditions sufficient for the production of a polypeptide of the invention in the culture and recovering said polypeptide of the invention from the culture;
- a **process for the production of a recombinant host cell** which produces a polypeptide of the invention comprising transforming or transfecting a host cell with the expression system of the invention such that the host cell, under appropriate culture conditions, produces a polypeptide of the invention; and
- a **recombinant host cell** produced by transforming or transfecting a host cell with the expression system of the invention such that the host cell, under appropriate culture conditions, produces a polypeptide of the invention.

For recombinant production, host cells may be genetically engineered to incorporate expression systems or portions thereof for a gene of the present invention.

Introduction of polynucleotides into host cells may be effected as appropriate, e.g. according to a conventional method, e.g. according to Davis et al. BASIC METHODS IN MOLECULAR BIOLOGY (1986); Sambrook et al. MOLECULAR CLONING: A LABORATORY MANUAL 2nd Ed., Cold Spring Harbor Laboratory Press, Cold Spring Harbor, N.Y. (1989), such as calcium phosphate transfection, DEAE-dextran mediated transfection, transvection, microinjection, cationic lipid-mediated transfection, electroporation, transduction, scrape loading, ballistic introduction or infection, or protein transduction. Appropriate host cells may be easily found. Examples of appropriate host cells include bacterial cells such as streptococci, staphylococci, E.coli, Streptomyces and Bacillus subtilis cells; fungal cells, such as yeast cells and Aspergillus cells; insect cells such as Drosophila S2 and Spodoptera Sf9 cells; isolated animal cells such as CHO, COS, HeLa, C127, CCL39, 3T3, BHK, HEK 293 and Bowes melanoma cells; and plant cells.

Appropriate expression systems include e.g. chromosomal, episomal and virus-derived systems, e.g. vectors derived from bacterial plasmids, bacteriophage, transposons, yeast episomes, insertion elements, yeast chromosomal elements, viruses, such as baculoviruses, papova viruses such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. An expression systems may contain control regions that regulate as well as engender expression. Generally, any system or vector suitable to maintain, propagate or express polynucleotides to produce a polypeptide in a host may be used. The appropriate nucleotide sequence may be inserted into an expression system as appropriate, e.g. according to a conventional method, e.g. according to Sambrook et al., MOLECULAR CLONING A LABORATORY MANUAL (supra).

For secretion of the translated protein into the lumen of the endoplasmic reticulum, into the periplasmic space or into the extracellular environment, appropriate secretion signals may be incorporated into the desired polypeptide. These may be heterologous signals.

If a polypeptide of the invention is to be expressed for use in screening assays, generally the polypeptide may be produced at the surface of the cell. In this event, the cells may be harvested prior to use in the screening assay. Alternatively the polypeptide may be produced within the cells.

A polypeptide of the invention may be recovered and purified from recombinant cell cultures as appropriate, e.g. according to a conventional method, including e.g. detergent extraction, ultracentrifugation, ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography, e.g. high performance liquid chromatography. If a polypeptide of the invention is denatured during isolation and/or purification, regeneration of the active conformation, e.g. refolding of a denaturated polypeptide of the invention, may be carried out as appropriate, e.g. according to a conventional method.

If a polypeptide of the invention is produced intracellularly, the cells may be e.g. lysed before the polypeptide is recovered.

A gene (fragment) of the invention or a polypeptide (fragment) of the invention may be used as a research reagent and material for the discovery of treatments and diagnostics to animal and human disease.

The transcription factor NF-kB plays a key regulatory role in regulating the expression of immune and inflammatory genes. One of the central components of the signal transduction pathway leading to NF-kB activation is IKK2. Proteins that interact with IKK2 can have modulatory function towards IKK2 activity. IKK2-55 was found to interact with IKK2 in yeast two hybrid screens and co-immunoprecipitation. It was found to modulate NF-kB activity as well as the activity of certain promoters. IKK2-55 as well as its pharmacological agonists or antagonists may therefore be used to treat a variety of immunological or inflammation-related diseases, including e.g. inflammation, arteriosclerosis, restenosis, reperfusion injury, inflammatory bowl disease, psoriasis, atopic dermatitis, transplant rejection, autoimmune diseases, allergy and cancer.

A **DINO** gene of the invention was found to belong to the HERC protein family typically containing a C-terminal HECT domain of about 350 amino acids and various numbers of RCC1 repeats at the N-terminal portion. HECT domain proteins constitute a subfamily of E3 ubiquitin ligases transfering ubiquitin from E2 conjugating enzyme to a specific substrate that is destined for degradation by the proteasome. Substrates for HERC family members have been shown to be involved in cell cycle control (cyclin E, p21 and p53), cancer (E6-AP) and in the regulation of TGF-13 family member BMP signaling (Mitsui et al. BBRC 266 [1999] 115-122; Cruz et al. FEBS letters 488 [2001] 74-80; Scheffner et al. Cell 75 [1993] 495-505; Zhu et al. Nature 400 [1999] 687-693). It was found by yeast two hybrid screening that DINO can interact with TSG101 gene. TSG101 modulates MDM2 degradation and MDM2/p53 feedback control by forming a TSG101/MDM2 regulatory loop (Li et al. PNAS 98 [2000] 1619-1624). In addition it has been found that, using reporter gene assays, DINO expression enhances p53 transcriptional activity. It can be expected that DINO is crucially involved in functional interaction between TSG101 and the MDM2/p53 loop and thus will be a mediator of cell proliferation, apoptosis and inflammatory responses.

DINO contains five RCC1 repeats at the N-terminus and differs from Cep1 which has 3 complete and half a repeat. RCC1 is a guanine nucleotide exchange factor (GEF) for the small GTPase Ran (Bischoff and Ponstingl Nature 354 [1991] 80-82). DINO may be expected to exert GEF activity on yet to identify small GTPases and may thus be involved in regulation of cell proliferation, cytosceletal morphology and intracellular transport.

DINO was found to be located as a cluster including Herc3 and Cep1 on chromosome 4.21-23.

A **LLR-J24-1 and -2 gene** was found to belong to the superfamily of type II transmembrane receptors with a CTLD in their extracellular part (Weis W.I. et al. Immunol. Rev. 163 [1998] 19-34). A significant part of these receptor genes are located in the NK receptor complex on human chromosome 12 (Sobanov, Y. et al. Immunogenetics 49 [1999] 99-105). The CTLDs of the receptors are typically encoded by three exons. The CTLDs developed during evolution from a C-type carbohydrate recognition domain. This contained a lectin-fold binding $Ca^{++}$ and had carbohydrate ligands such as the hepatic asialoglycoprotein receptor. The binding domain of an LLR-J24 gene has the modified characteristics of a CTLD, e.g. it can fold independently of $Ca^{++}$ and bind non-carbohydrate ligands. To establish the relationship of the LLR-J24 gene to the other CTLD family genes the predicted amino acid sequences of the CTLDs were compared. The obtained data display that LLR-J24 forms together with LOX-1, CLEC-1 and CLEC-2 a subfamily of closer related lectin-like receptors of the NKC. The highest similarity LLR-J24 was seen with LOX-1 (44 %), CLEC-1 and CLEC-2 are somewhat more distantly related (37 %). The subfamily of CTLD NK receptors consisting of the NKG2 and the CD94 proteins are the next closest related proteins (between 28 % for NKG2-E and 33 % for NKG2-D). The AICL/LLT-1/CD69 group and the KLRF1, MAFA and NKR-P1 receptors are again somewhat more distant and are displayed in separate branches in a multiple alignment.

LOX-1 has been found to be expressed by vascular endothelial cells and macrophages. It is dynamically regulated in vivo by inflammatory stimuli such as TNF-alpha, lipopolysaccharide and oxLDL, but also by TGF-β, angiotensin II and by fluid shear stress in hypertension. Importantly, LOX-1 has been found to be up-regulated in atherosclerotic lesions where it is believed to contribute to foam cell formation and endothelial dysfunction. It was described to bind oxLDL and anionic phospholipids as well as aged red blood cells and apoptotic cells (Sawamura T. et al. Nature 386 [1997] 73-77). One of the earliest events in aged or apoptotic cells is the exposure of phosphatidylserine on the outside of the plasma membrane, which is recognized and bound by LOX-1. It has therefore been proposed that LOX-1 is involved in the removal of those cells from the blood stream. In contrast to the signalling events initiated upon ligand binding of other lectin-like receptors such as the CD94/NKG2 receptors, molecules and cellular fragments bound to LOX-1 are internalized by endocytosis.

Similar to LOX-1, the LLR-24J genes can be expected to bind oxidized lipoproteins such as oxLDL, parasite surface structures and apoptotic cell fragments.

The other two closely related genes are named CLEC-1 and CLEC-2, the corresponding cDNAs have recently been identified in EST databases and described (Colonna M. et al. Eur. J. Immunol. 30 [2000] 697-704). CLEC-1 mRNA was shown to be preferentially expressed in DCs and was also found in placenta, lung and thymus. CLEC-2 mRNA was also detected in DCs, but in addition showed wider expression in the hematopoietic lineage. CLEC-2 mRNA levels were furthermore especially high in liver. No ligands have yet been identified for CLEC-1 and CLEC-2.

The next closest related genes are the NKG2 NK receptor genes. It is now well established that the lectin-like NK receptors possess important roles for monitoring proper MHC class I expression according to the missing-self hypothesis (Hoglund P. et al. Immunol. Rev. 155 [1997] 11-28). In human NK cells the CD94 chain forms heterodimeric receptors with different NKG2 isoforms, which can bind the non-classical MHC class Ib molecule HLA-E (Lopez-Botet M. et al. Semin. Immunol. 12 [2000] 109-119). CD94/NKG2A functions as an inhibitory receptor, the CD94/NKG2C receptor can activate NK cells. HLA-E predominantly presents peptides from the leader sequences of other class I molecules and is only transported to the surface when loaded with the appropriate peptides. Thus recognition of HLA-E by the CD94/NKG2A receptor is a sensitive mechanism to monitor biosynthesis of MHC class I molecules, which can be down-modulated by many viruses. The function of the activating CD94/NKG2C receptor in this context is still not fully understood. The activating NKG2D receptor, only distantly related to the other NKG2 molecules, forms homodimers and recognizes the class I-like molecule MICA and MICB, which is upregulated by stress, e.g. viral infection, and on some tumor cells (Bauer S. et al. Science 285 [1999] 727-729). Additional ligands are the so-called UL16 binding proteins, which bind the CMV-encoded UL16 protein and are also upregulated on tumor cells. NKG2-D is therefore an activating receptor directly related to NK-mediated killing of virus-infected and transformed cells. It is noteworthy that among the NKG2 proteins LLR-J24 is closest related to NKG2-D. The corresponding CD94 and NKG2 proteins in mice appear to have comparable functions. Furthermore, in mice over fourteen isoforms of Ly49 receptors are involved in monitoring expression of different MHC class I molecules.

Similar to the NKG2 receptors it can be expected that LLR-J24 can bind to certain MHC class I /peptide complexes or to class I-related molecules up-regulated on virally infected and tumor cells.

The sequence of the LLR-J24 genes displays high similarity to the recently described murine DECTIN-1 cDNA with the exception that a stalk exon was missing from the first isolated human cDNA. It could be determined that this is found correctly spliced in a subfraction of about 5 to 10 % of the transcripts (**Figures 9 and 10**), the corresponding predicted full-length protein sequences of mouse and human DECTIN-1 were compared. Both sequences show an identity of 59 % (similarity 69 %). The high similarity is conserved in the cytoplasmic and extracellular domains including the stalk domain. Close to the N-terminus of the cytoplasmic domain an ITAM-like sequence is present in the human as well as the murine versions. This sequence differs slightly from a classical ITAM motif as it is found in the CD3-γ chain or the DAP12 molecule (Lanier L. L. et al. Nature 391 [1998] 703-707). The first tyrosine (residue 3) in the motif is 4 amino acid positions before the leucine (residue 7), whereas this distance is 3 amino acid positions in the classical $Yx_2Lx_{6-8}Yx_2L$ motif. The following residues of the motif are correctly placed. Both proteins further show consensus sites for N-glycosylation. The lectin-like domain of the human and murine versions lack most of the residues described to be involved in $Ca^{++}$-binding suggesting a non-carbohydrate ligand. The transmembrane domain does not display a charged amino acid indicating that association with ITAM-containing adapters such as the DAP molecules does not take place.

The expression of LLR-J24 mRNA in primary human DCs, monocytes, T and B lymphocytes as well as endothelial cells was also investigated. The data obtained show that human LLR-J24 mRNA is abundantly and selectively expressed in monocyte-derived DCs (MoDC) and in DCs derived from $CD34^+$ hematopoietic progenitor cells (HPDC). A small amount of LLR-J24 mRNA is present in primary monocytes, whereas primary T and B lymphocytes as well as endothelial cells, treated or untreated with different stimuli, does not detectably express LLR-J24 transcripts. No expression is visible in Northern blots including several additional cell lines, i.e. Jurkat, RPMI8866, NKL and NK92. The size of the RNA in DCs was estimated to be 3.0 to 3.5 kb from the Northern blots, which corresponds to the expected transcript sizes assuming the use of the first or several consecutive polyadenylation signals detected in the gene in a region 1.6 to 1.9 kb downstream from the stop codon.

Activation and final maturation of DCs is associated with altered expression of genes that regulate their migration and function. It was therefore tested whether stimulation of DCs by inflammatory mediators, CD40-ligand or zymosan A would lead to a change in expression levels for LLR-J24 mRNA. As shown in **Figure 8**, treatment of MoDC with a combination of IL-1$\beta$ and TNF-$\alpha$ for 18 to 24 hours resulted in a reproducible, three to five-fold reduction of LLR-J24 mRNA. Alternatively, HPDC were activated by mAb specific for the CD40 receptor in the absence or presence of zymosan A stimulating phagocytosis of the yeast particles. Based on these results, it appears that downmodulation of LLR-J24 mRNA is specially pronounced in cultures treated with strong inducers of maturation such as TNF-$\alpha$ and IL-1$\beta$ or CD40 ligation combined with phagocytosis stimulation. Since MDC chemokine (CCL22) is known to be strongly induced in DCs upon their maturation, the same Northern blot was hybridized with a specific probe for MDC to confirm successful activation of MoDC or HPDC. As seen in **Figure 8**, a strong induction of MDC parallels downregulation of LLR-J24 transcripts in both types of DCs.

Since LLR-J24-1 lacked the region of the stalk exon, the presence of the stalk exon in the LLR-J24 transcripts from DCs was investigated. Northern blot analysis was unable to distinguish transcripts which were predicted to differ by only 141 bases. Therefore reverse transcription PCR was performed using a primer pair to amplify a fragment between the second and fourth exon. Two products of 505 bp and 367 bp were obtained corresponding in size to the predicted fragments with and without the stalk exon, respectively (**Figures 9** and 10). It is estimated that approximately 5 to 10 % of the products were of the size containing the stalk exon. This upper band was excised from the gel, subcloned and two of the obtained clones sequenced. Both clones displayed correctly spliced stalk exons as predicted from the genomic sequence. The PCR fragments obtained with primer pairs were further analyzed, which amplify fragments of the mRNA from the 5'-untranslated region to the stalk exon and from the stalk exon to the sixth exon, respectively. In both cases the major obtained products corresponded to the sizes predicted for the correctly spliced exons. These data show that a fraction of the LLR-J24 transcripts contained the stalk exon and was also correctly spliced at the other exons.

The type II lectin-like receptors have been shown to usually dimerize and to form either homodimers or heterodimers combining two different receptor chains. In certain cases the dimerization with a heterologous lectin-like receptor chain has been shown to be necessary for surface expression. This is, for example, the case for the NKG2/CD94 receptors (Carretero M. et al. Eur J Immunol. 27 [1997] 563-567. Therefore, the surface expression of FLAG-tagged recombinant LLR-J24 in 293 cells and HUVEC following transient transfection with the respective expression constructs were evaluated. To evaluate surface expression, staining of non-permeabilized cells with an anti-FLAG antibody were compared to parallel samples of cells which were permeabilized with Triton X-100 before the staining procedure. For LLR-J24 constructs were used with and without the stalk exon. Full-length LLR-J24 efficiently accumulated at the cell surface of HUVEC as shown by strong staining of non-permeabilized cells (**Figure 6A**). In contrast, no detectable levels of the LLR-J24 isoform without stalk were visible on non-permeabilized cells, although significant intracellular staining of permeabilized cells was obtained. A closer analysis of permeabilized cells at higher magnification revealed that cells transfected with full-length LLR-J24-2 expression constructs display strong staining of peri-nuclear cellular compartments as well as surface expression visible over the whole cell **(Figure 6B).** The stalkless LLR-J24-1 appears to be homogenously distributed throughout the cytoplasm, but is not appearing at the surface. Similar results were obtained with 293 cells (data not shown).

In a further aspect the invention provides:

- an isolated **polynucleotide encoding** the transmembrane **protein LLR-J24;**
- an isolated **polynucleotide encoding** the **extracellular domain of LLR-J24**;
- an isolated **polypeptide** which is the **extracellular domain of LLR-J24;** and
- an isolated **polypeptide** which is the transmembrane **protein LLR-J24**.

The invention also provides the **use** of a gene of the invention **as a diagnostic reagent**.

Detection of a mutated form of a gene of the invention associated with a dysfunction will provide a diagnostic tool, e.g. in a diagnostic assay, that may add to or define a diagnosis of a disease or susceptibility to a disease which results from under-expression, over-expression or altered expression of the corresponding gene or a mutant version therof, and/or a polypeptide of the invention. Individuals carrying mutations in the corresponding gene may be detected at the DNA level according to a conventional method.

Nucleic acids for diagnosis may be obtained from a subject's cells, such as from blood, urine, salvia, tissue biopsy or autopsy material. The genomic DNA may be used directly for detection or may be amplified enzymatically by using PCR or other amplification techniques prior to analysis. RNA or cDNA may also be used in the analysis similarly. Deletions and insertions may be detected by a change in size of the amplified product in comparison to the normal genotype. Point mutations may be identified by hybridizing amplified DNA to labeled gene nucleotide sequences of the invention. Perfectly matched sequences may be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing, e.g. according to Myers et al. Science 230 (1985) 1242. Sequence changes at specific locations may also be revealed by nuclease protection assays, such as RNase and S 1 protection or the chemical cleavage method, e.g. according to Cotton et al. Proc. Natl Acad. Sci. USA 85 (1985) 4397-4401. An array of oligonucleotides probes comprising the gene nucleotide sequence of the invention or fragments thereof may be constructed to conduct efficient screening of e.g. genetic mutations. Array technology methods may e.g. be used to address a variety of questions in molecular genetics including gene expression, genetic linkage, and genetic variability, e.g. according to M. Chee et al. Science 274 (1996) 610-613.

In case of an IKK2-55 gene of the invention a diagnostic assay offers a process for diagnosing or determining a susceptibility to diseases, such as immunological or inflammation-related diseases, including e.g. inflammation, arteriosclerosis, restenosis, reperfusion injury, inflammatory bowel disease, psoriasis, atopic dermatitis, transplant rejection, autoimmune diseases, allergy and cancer; through detection of mutation in a gene of the invention.

In case of a DINO gene of the invention a diagnostic assay offers a process for diagnosing or determining a susceptibility to diseases, e.g. including inflammatory diseases, such as acute or chronic inflammatory diseases, autoimmune diseases, transplant rejection, cancer, atherosclerosis and restenosis.

In case of a LLR-J24 gene of the invention a diagnostic assay offers a process for diagnosing or determining a susceptibility to diseases related to unbalanced, increased or decreased DC functions, including e.g. chronic inflammatory diseases, autoimmune diseases, transplant rejection crisis, inflammatory skin diseases such as contact hypersensitivity and atopic dermatitis, or virally induced immune suppression, e.g. AIDS, e.g. all forms of suceptibility to viral, bacterial, parasite infectious diseases and cancer.

Diseases may be diagnosed e.g. according to a conventional method, e.g. by methods comprising determining from a sample derived from a subject an abnormally decreased or increased level of a gene or polypeptide or gene mRNA of the invention. Decreased or increased expression can be determined at the RNA level e.g. according to a conventional method for the quantitation of polynucleotides, such as, for example, PCR, RT-PCR, RNase protection, Northern blotting and other hybridization methods. Assay techniques that may be used to determine levels of a protein, such as a polypeptide of the invention, in a sample derived from a host may be carried out e.g. according to a conventional method as conventional. Such assay techniques include e.g. radioimmunoassays, competitive-binding assays, Western Blot analysis and ELISA assays.

Thus in a further aspect, the invention provides a **diagnostic kit** for a disease or susceptibility to a disease, e.g. such as mentioned above; comprising as a main component

a) a gene of the invention, including e.g. allelic variants thereof, or a fragment thereof; or a splice variant thereof;
b) a nucleotide sequence complementary to that of a);
c) a polypeptide of the invention, including e.g. a polypeptide of an amino acid sequence which has at least 80 % identity thereto, e.g. including a fragment or a variant of a polypeptide of the invention, or a fragment or a variant of a polypeptide of an amino acid sequence which has at least 80 % identity to said polypeptide of the invention, or
d) an antibody to a polypeptide of the invention;

e.g. any such kit, a), b), c) or d) may comprise a substantial component, e.g. including an appropriate environment of a sample to be tested, e.g. appropriate means to determine the effect of any of a), b), c) or d) in a sample to be tested.

A gene according to the invention is also useful for chromosome identification. The sequence is specifically targeted to and can hybridize with a particular location on an individual human chromosome. The mapping of relevant sequences of the invention to chromosomes is an important first step in correlating those sequences with gene associated disease. Once a sequence has been mapped to a precise chromosomal location, the physical position of the sequence on the chromosome may be correlated with genetic map data.

Corresponding data is disclosed in e.g. V. McKusick Mendelian Inheritance in Man (available on line through Johns Hopkins University Welch Medical Library). The relationship between genes and diseases that have been mapped to the same chromosomal region may be identified through linkage analysis (coinheritance of physically adjacent genes). The differences in the cDNA or genomic sequence between affected and unaffected individuals may also be determined. If a mutation is observed in some or all of the affected individuals but not in any normal individuals, then the mutation is likely to be the causative agent of the disease.

Thus e.g. IKK2-55 localizes to chromosome 12q23.1, in close proximity to the long form of APAF1 (apoptotoc protease activating factor 1), and the LLR-J24 gene of the invention has been used for chromosome identification on the short arm of chromosome 12 in the NK receptor gene complex; susceptibilities to viral diseases and arthritis have been mapped to similar syntenic regions in rodents.

A polypeptide of the invention or fragment thereof, or cells expressing such polypetide of the invention or fragment thereof, can also be used as immunogens to produce antibodies immunospecific for said polypeptide of the invention. The term "immunospecific" means that the antibodies have substantially greater affinity for the said polypeptide of the invention than their affinity for other related or unrelated polypeptides.

Antibodies generated against a polypeptide of the invention may be obtained e.g. by administering such polypeptide, or an epitope-bearing fragment-analogue or cell, to an animal, preferably a non-human, using routine protocols. For preparation of monoclonal antibodies, an appropriate technique which provides antibodies, e.g. produced by continuous cell line cultures, may be used, e.g. the hybridoma technique (Kohler, G. and C. Milstein Nature 256 [1975] 495-497), the trioma technique, the human B-cell hybridoma technique (Kozbor et al. Immunology Today 4 [1983] 72) and the EBV-hybridoma technique (Cole et al., MONOCLONAL ANTIBODIES AND CANCER THERAPY, Alan R. Liss, Inc.[1985] 77-96). Techniques for the production of single chain antibodies (e.g. U.S. Pat. No. 4,946,778) can also be adapted to produce single chain antibodies to a polypeptide of the invention. Also, transgenic mice, or other organisms including other mammals, may be used to express humanized antibodies. Antibodies as described above may be used e.g. in the isolation or in the identification of a clone expressing a polypeptide according to the invention or for the purification of a polypeptide (fragment) of the invention by affinity chromatography.

Antibodies against a polypeptide of the invention may also be useful in the treatment of diseases, e.g. in case of antibodies against a DINO gene, in diseases such as inflammatory diseases, e.g. acute or chronic inflammatory diseases, autoimmune diseases, transplant rejection, cancer, atherosclerosis and restenosis; or in the case of antibodies against a LLR-J24 gene, in disaeses such as chronic inflammatory diseases, autoimmune diseases, transplant rejection crisis, including e.g. inflammatory skin diseases such as contact hypersensitivity, atopic dermatitis, or virally induced immune suppression, e.g. AIDS, e.g. all forms of suceptibility to viral, bacterial, parasite infectious diseases and cancer.

In a further aspect the invention provides an **antibody** against a polypeptide of the present invention. An antibody of the invention includes a bispecific antibody binding to the LLR-J24 polypeptide and a viral, bacterial or eukaryotic parasite or to a cancer cell.

In a further aspect the invention provides a bispecific antibody binding to the LLR-J24 polypeptide and a viral, bacterial or eukaryotic parasite.

In a further aspect the invention provides a bispecific antibody binding to the LLR-J24 polypeptide and a cancer cell.

In place of a bispecific antibody another bispecific reagent having the ability to bind to the LLR-J24 polypeptide and a viral, bacterial or eukaryotic parasite or to a cancer cell may also be used.

In a further aspect the invention provides a **bispecific reagent** which is not an antibody and having the ability to bind to the LLR-J24 polypeptide and a viral, bacterial or eukaryotic parasite or to a cancer cell.

In a further aspect the invention provides a **method of inducing an immunological response** in a mammal which comprises delivering a polypeptide of the invention via a vector, directing expression of a corresponding gene of the invention in vivo in order to induce such an immunological response to produce antibodies to protect said animal from diseases; and, in a yet further aspect, an immunological/vaccine formulation (composition) which, when introduced into a mammalian host, induces an immunological response in that mammal to a polypeptide of the invention, wherein the composition comprises a polypeptide of the invention or a gene of the invention.

In a further aspect the invention provides a method for inducing an immunological response in a mammal which comprises inoculating the mammal with a DINO polypeptide of the invention, or a fragment thereof, or with a LLR-J24 polypeptide of the invention, or a fragment thereof, respectively, adequate to produce antibody and/or T cell immune response to protect said animal from diseases, e.g. diseases as indicated above, e.g. wherein a LLR-J24 polypeptide of the invention is preferably of **SEQ ID NO:6** or **SEQ ID NO:8,** more preferably of **SEQ ID NO:8.**

A vaccine formulation may further comprise a suitable carrier. Since a polypeptide of the invention may be broken down in the stomach, it may be preferably administered parenterally (including subcutaneous, intramuscular, intravenous, intradermal etc. injection). Immunological/vaccine formulations suitable for parenteral administration include e.g. aqueous and non-aqueous sterile injection solutions which may contain anti-oxidants, buffers, bacteriostats and solutes which render the formulation isotonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of a sterile liquid carrier immediately prior to use. The vaccine formulation may also include adjuvant systems for enhancing the immunogenicity of the formulation, such as oil-in water systems and other appropriate systems. The dosage will depend on the specific activity of the vaccine and can be readily determined by routine experimentation.

A polypeptide of the invention may be responsible for many biological functions, including those underlying many pathological states. Accordingly, it is desirable to find compounds and drugs which activate, e.g. biocompetantly, e.g. transcriptionally and/or stimulate activity of a polypeptide of the invention, or expression of a gene of the invention on the one hand (agonists) and which can inhibit the function of a polypeptide of the invention or expression of a gene of the invention on the other hand (antagonists). A polypeptide of the invention or functional mimetics thereof, e.g. according to Coligan et al., Current Protocols in Immunology 1(2) Chapter 5 (1991), may thus be used to assess the binding of agonists or antagonists of the receptor polypeptide of the invention, e.g. in cells, cell-free preparations, chemical libraries, and natural product mixtures, e.g. in a screening assay.

Agonists and antagonists of a IKK2-55 polypeptide of the invention may be used in the treatment of diseases including e.g. immunological or inflammation-related diseases, such as inflammation, arteriosclerosis, restenosis, reperfusion injury, inflammatory bowel disease, psoriasis, atopic dermatitis, transplant rejection, autoimmune diseases, allergy and cancer.

Agonists and antagonists of a DINO polypeptide of the present invention may be used in the treatment of diseases including e.g. inflammatory diseases, acute or chronic, autoimmune diseases, transplant rejection, cancer, atherosclerosis and restenosis.

Agonists and antagonists of a LLR-J24 polypeptide of the invention may be used in the treatment of diseases including e.g. chronic inflammatory diseases, autoimmune diseases, transplant rejection crisis, such as inflammatory skin diseases, e.g. contact hypersensitivity, atopic dermatitis, or virally induced immune suppression, e.g. AIDS, e.g. all forms of suceptibility to viral, bacterial, parasite infectious diseases, and cancer.

Stimulation or inhibition of expression of a gene of the invention, e.g. by Low Molecular Weight compounds (LMWs) or antisense oligonucleotides, may be desirable to modulate the effects of a corresponding polypeptide of the invention and its ligands on the physiology/function of the EC, or DC, respectively.

Screening procedures may involve the production of appropriate cells which express the receptor of a polypeptide of the invention on the surface thereof in the case of IKK2-55 or LLR-J24. Appropriate cells include cells e.g. from mammals, yeast and Drosophila. In the case of IKK2-55, cells expressing IKK2-55; and in the case of LLR-J24, cells expressing the receptor (or cell membranes containing the expressed receptor), may be contacted with a test (candidate) compound to observe binding, or stimulation or inhibition of a functional response.

A screening assay may be used to test binding of a candidate compound wherein binding to the cells bearing the IKK2-55, or bearing the LLR-J24 receptor, respectively, may be detected by means of a label directly or indirectly associated with the candidate compound or in an assay involving competition with a labeled competitor. A screening assay may be used further to test whether the candidate compound results in a signal generated by activation of the receptor, using detection systems appropriate to the cells bearing the IKK2-55, or the LLR-J24 receptor, respectively, at their surfaces. Inhibitors of activation may be assayed in the presence of a known agonist and the effect on activation by the agonist by the presence of the candidate compound may be observed.

A screening assay may comprise the steps of mixing a candidate compound with a solution containing a polypeptide of the invention to form a mixture, determining activity of said polypeptide in the mixture, and comparing the activity of the mixture with the activity of a standard.

A gene (cDNA) of the invention, a polypeptide of the invention and antibodies to a polypeptide of the invention may also be used to provide a screening assay for detecting the effect of candidate compounds on the production of said gene (mRNA) and said polypeptide in cells. For example, an ELISA may be constructed for determining cell associated levels of said polypeptide, e.g. using monoclonal and polyclonal antibodies according to a conventional method, and that ELISA may be used to discover agents (agonists or antagonists) which may inhibit (antagonist) or enhance (agonist) the production or the activity of said polypeptide in suitably manipulated cells or tissues. An assay for screening may be conducted e.g. according to a conventional method.

Examples of potential (ant)agonists of a gene of the invention include antibodies or, in some cases, oligonucleotides or protein (polypeptides) closely related to the ligand (antagonist bound to a polypeptide of said gene) of said gene, e.g. a fragment of said ligand, or small molecules, which bind to the receptor but do not elicit a response, so that the activity of the receptor is prevented.

Examples of potential (ant)agonists according to the invention include compounds which bind to a polypeptide according to the invention, including e.g. oligopeptides, polypeptides, protein, antibodies, mimetics and small molecules, e.g. low molecular weight compounds (LMW's).

Thus in a further aspect, the invention provides a **screening assay for identifying an agonist or an antagonist** of a polypeptide of the invention which assay comprises as a main component

a) a polypeptide of the invention,
b) a recombinant cell expressing a polypeptide of the invention,
c) a cell membrane expressing a polypeptide of the invention, or

d) an antibody to a polypeptide of the invention;

and means for a contact with a candidate compound; e.g. means for determining the effect of the candidate compound on any of a), b), c) or d);
e.g., determining whether in the presence of the candidate compound there is a decrease or enhancement in the production and or the biological activity of a polypeptide according to the invention, e.g. by comparison of the activity of any of a), b), c) or d) in the presence and in the absence of the candidate compound; and
in another aspect, a **method of identifying an agonist or antagonist,** preferably agonist, including e.g. ligands, receptors, antibodies or LMW's, which decreases or enhances the production and/or the biological activity of a polypeptide of the invention, which method comprises

A) contacting

a) a polypeptide of the invention,
b) a recombinant cell expressing a polypeptide of the invention,
c) a cell membrane expressing a polypeptide of the invention, or
d) an antibody to a polypeptide of the invention

with a candidate compound;
B) determining the effect of the candidate compound on any of a), b), c) or d); e.g. determining whether in the presence of the candidate compound there is a decrease or enhancement in the production and or the biological activity of a polypeptide of the invention, e.g. by comparison of the activity of any of a), b), c) or d) in the presence and in the absence of the candidate compound; and
C) choosing an agonist or antagonist determined in step B),
e.g. choosing an appropriate candidate compound from which an agonist/antagonist effect is positively determined in step B).

In any such screening assay, a), b), c) or d) may comprise a substantial component. A candidate compound includes compound (libraries), from which the effect on any of a), b), c) or d) is unknown. Compound (libraries) include compounds which are set out above as (ant)agonists to a polypeptide according to the invention. An (ant)agonist is a candidate compound from which an effect on any of a), b), c) or d) has been found in a screening assay or in a method for identifying (ant)agonists as described above. An (ant)agonist may decrease or enhance the production and or the biological activity of a polypeptide according to the invention.

In a further aspect the invention provides **an antagonist or an agonist,** preferably an antagonist, of a polypeptide of the invention, which is characterized in that said antagonist or agonist can be provided by the following method steps:

A) contacting

a) a polypeptide of the invention,
b) a recombinant cell expressing a polypeptide of the invention,
c) a cell membrane expressing a polypeptide of the invention, or
d) an antibody to a polypeptide of the invention

with a candidate compound;
B) determining the effect of said candidate compound on any of a), b), c) or d), e.g. determining whether in the presence of the candidate compound there is a decrease or enhancement in the production and or the biological activity of a polypeptide of the invention; e.g. by comparison of the activity of any of a), b), c) or d) in the presence and in the absence of the candidate compound; and
C) choosing an agonist or antagonist determined in step B),
e.g. choosing an appropriate candidate compound from which an agonist/antagonist effect is positively determined in step B).

An (ant)agonist of IKK2-55 (polypeptide) of the invention may be used in the treatment of diseases, e.g. including immunological or inflammation-related diseases, such as inflammation, arteriosclerosis, restenosis, reperfusion injury, inflammatory bowel disease, psoriasis, atopic dermatitis, transplant rejection, autoimmune diseases, allergy and cancer. An (ant)agonist of DINO (polypeptide) of the invention may have immune modulatory activities and may be used in the treatment of diseases including e.g. inflammatory diseases, such as acute or chronic inflammatory diseases, autoimmune diseases, transplant rejection, cancer, atherosclerosis and restenosis.

An (ant)agonist of LLR-24 (polypeptide) of the invention may have immune modulatory activities and may be used in the treatment of diseases including e.g. chronic inflammatory diseases, autoimmune diseases, transplant rejection crisis, such as inflammatory skin diseases, e.g. contact hypersensitivity, atopic dermatitis, or virally induced immune suppression, e.g AIDS, e.g. all forms of suceptibility to viral, bacterial, parasite infectious diseases and cancer. An (ant) agonist of a polypeptide of the invention may thus be useful as a pharmaceutical.

For such use several approaches are available:

If the activity of a gene and/or polypeptide of the invention is in excess, one approach comprises administering to a subject an antagonist of a polypeptide of the invention, e.g. in combination with a pharmaceutically acceptable excipient, in an amount effective to inhibit activation of a gene and/or polypeptide of the invention by blocking binding of ligands to said polypeptide, or by inhibiting a second signal, and thereby alleviating the abnormal condition caused by e.g. over-, under- or altered expression of said gene (or a mutant version therof). In another approach, e.g. in case of DINO or LLR-J24, soluble forms of a corresponding polypeptide of the invention, still capable of binding the ligand in competition with endogenous polypeptide, may be administered. Typical embodiments of such competitors may comprise fragments of said polypeptide of the invention.

In a further aspect the invention provides a **soluble LLR-J24 polypeptide** of the invention which binds to a ligand on T cells or other immune cells and which is able to activate the immune function and e.g. anergy.

In a yet further approach, the invention comprises **inhibition of expression of the gene** encoding endogenous polypeptide of the invention using expression blocking techniques.

Known such techniques involve the use of antisense sequences, either internally generated or separately administered, e.g. according to O'Connor J. Neurochem. 56 (1991) 560 in Oligodeoxynucleotides as Antisense Inhibitors of Gene Expression, CRC Press, Boca Raton, Fla. (1988). Alternatively, oligomers, e.g. oligonucleotides which form triple helices with a gene according to the invention may be supplied, e.g. according to Lee et al. Nucleic Acids Res. 6 (1979) 3073; Cooney et al. Science 241 (1988) 456; and Dervan et al. Science 251 (1991) 1360. Such oligomers may be administered per se or may be expressed in vivo.

For treating abnormal conditions related with an under-expression of a polypeptide of the invention and its activity, one approach comprises administering to a subject in need of an increased expression of a polypeptide of the invention a therapeutically effective amount of a compound which activates a gene of the invention (agonist), e.g. in combination with a pharmaceutically acceptable excipient, to thereby alleviate the abnormal condition.

Alternatively, gene therapy may be employed to effect the endogenous production of a polypeptide of the invention by the relevant cells in the subject. For example, a gene of the invention may be engineered for expression in a replication defective retroviral vector, e.g. according to a method as indicated above. A retroviral expression construct obtained may be isolated and introduced into a packaging cell transduced with a retroviral plasmid vector containing RNA encoding a polypeptide corresponding to said gene of the invention, such that the packaging cell produces infectious viral particles containing the gene of interest. These producer cells may be administered to a subject for engineering cells in vivo and expression of the polypeptide in vivo. For overview of gene therapy, see e.g. Chapter 20, Gene Therapy and other Molecular Genetic-based Therapeutic Approaches (and references cited therein) in Human Molecular Genetics, T Strachan and A P Read, BIOS Scientific Publishers Ltd. (1996).

In another aspect the invention provides **an agonist or an antagonist,** preferably an antagonist, of the polypeptides of the invention **for use as a pharmaceutical;** e.g. for the treatment or prevention of diseases as indicated above, and, in another aspect:

- **the use of an agonist or antagonist of the invention in the preparation of a medicament** for the treatment or prevention of diseases as indicated above; and
- a **soluble form** of a IKK2-55 polypeptide, or of a LLR-J24 polypeptide, respectively, of the invention **for use as a pharmaceutical**, e.g. for the treatment of the same diseases wherein an IKK2-55, or LLR-J24, respectively, (ant) agonist of the invention is suitable.

An (ant)antagonist of a polypeptide according to the invention may be administered in the form of a pharmaceutical composition.

In another aspect the invention provides a **pharmaceutical composition** comprising an agonist or an antagonist, preferably an antagonist, of the invention as an active ingredient in combination with pharmaceutically acceptable excipient(s)/carrier(s); e.g. a pharmaceutical composition wherein that said antagonist or agonist can be provided by the following method steps:

A) contacting

a) a polypeptide of the invention,
b) a recombinant cell expressing a polypeptide of the invention,
c) a cell membrane expressing a polypeptide of the invention, or
d) an antibody to a polypeptide of the invention

with a candidate compound,
B) determining the effect of said candidate compound on any of a), b), c) or d); for example, determining whether in the presence of the candidate compound there is a decrease or enhancement in the production and/or the biological activity of a polypeptide of the invention, e.g. by comparison of the activity of any of a), b), c) or d) in the presence and in the absence of the candidate compound; and
C) choosing an agonist or antagonist determined in step B); for example, choosing an appropriate candidate compound from which an agonist/antagonist effect is positively determined in step B).

Such pharmaceutical composition may be produced as appropriate, e.g. according to a conventional method, e.g. by mixing an (ant)agonist provided by the method steps A), B) and C) with excipient(s)/carrier(s) and further processing the mixture obtained, to obtain a pharmaceutical composition for appropriate administration.

In a further aspect the invention provides a **method of treating abnormal conditions related to both an excess of or an insufficient level,** preferably an excess, **of expression** of a gene of the invention; or related to both an excess and insufficient activity of a polypeptide of the invention, preferably an excess; e.g. in case of IKK2-55 a method of treating diseases, including immunological or inflammation-related diseases, such as inflammation, arteriosclerosis, restenosis, reperfusion injury, inflammatory bowel disease, psoriasis, atopic dermatitis, transplant rejection, autoimmune diseases, allergy and cancer; e.g. in case of DINO a method of treating diseases, including inflammatory diseases, such as acute or chronic inflammatory diseases, autoimmune diseases, transplant rejection, cancer, atherosclerosis and restenosis; e.g. in case of LLR-24 a method of treating diseases, including chronic inflammatory diseases, autoimmune diseases, transplant rejection crisis, such as inflammatory skin diseases, e.g. contact hypersensitivity, atopic dermatitis, or virally induced immune suppression, e.g. AIDS, e.g. all forms of suceptibility to viral, bacterial, parasite infectious diseases and cancer; comprising administering a therapeutically effective amount of an agonist or antagonist of the invention, which can for example be provided by the method steps A), B) or C) as indicated above, e.g. in combination with pharmaceutically acceptable excipient(s)/carrier(s); e.g. in case of IKK2-55 or LLR-J24, respectively, administering a therapeutically effective amount of a soluble form of a IKK2-55, or LLR-J24, respectively, polypeptide of the invention, e.g. in combination with pharmaceutically acceptable excipient(s)/carrier(s); to a subject in need of such treatment.

In another aspect the invention provides a **method of treating infection or cancer** by administering an effective amount of a bi-specific antibody or reagent according to the invention which is able to increase antigen-uptake and presentation and activation of T cells by DCs, to a subject in need of such treatment.

In another aspect the invention provides a method of treating infection or cancer comprising administering an effective amount of a soluble LLR-J24 protein which is able to activate the immune function to a subject in need of such treatment.

IKK2-55 is an intracellular protein. Preferred forms of systemic administration of a pharmaceutical composition of the invention include injection, typically by intravenous injection. Other injection routes, such as subcutaneous, intramuscular, or intraperitoneal, may be used. Alternative means for systemic administration include transmucosal and transdermal administration, e.g. using penetrants such as bile salts or fusidic acids or other detergents. In addition, if properly formulated in enteric or encapsulated formulations, oral administration may also be possible. Administration of a composition according to the invention may also be topical and/or localized, e.g. in the form of cremes, pastes, gels and the like. The dosage range required may depend on the choice of a polypeptide, or an agonist or antagonist of the invention, the route of administration, the nature of the pharmaceutical composition, the nature of the subject's condition, and the judgment of the attending practitioner. Suitable dosages, however, may be in the range of from about 0.1 mg/kg to about 10 mg/kg, such as from about 0.1 mg/kg to about 1 mg/kg of subject. Variations in the needed dosage, however, may be expected in view of the variety of compounds available and the differing efficiencies of various routes of administration. For example, oral administration would be expected to require higher dosages than administration by intravenous injection. Variations in these dosage levels may be adjusted, e.g. according to standard empirical routine for optimization.

Polypeptides used in treatment may also be generated endogenously in a subject who is e.g. in need of such treatment, in treatment modalities often referred to as "gene therapy", e.g. as described above. Thus, for example, cells from a subject may be engineered ex vivo with a polynucleotide, such as a DNA or RNA, to encode a IKK2-55 polypeptide e.g. by use of a retroviral plasmid vector. Engineered cells may be introduced into the subject who is e.g. in need of such treatment.

According to the invention genetically modified DCs may be provided which express LLR-J24 constructs which are able to improve antigen-uptake and presentation, e.g. which are able to improve T cell and immune system activation.

In another aspect the invention provides **genetically modified dendritic cells** which express LLR-J24 constructs which are able to improve antigen-uptake and presentation, e.g. which are able to improve T cell and immune system activation.

An antibody, a ligand or a reagent binding to a LLR-J24 polypeptide may inhibit DC formation/maturation, and in consequence may e.g. improve immune function and anergy.

In another aspect the invention provides **an antibody, a ligand or a reagent binding to the LLR-J24 polypeptide** of the invention, which inhibits DC formation/maturation.

**Description of the Figures:**

**Figure 1 (IKK2-55)**

Interaction by Co-IP of IKK2 with IKK2-55. Flag-tagged IKK2-55 and myc-tagged IKK2 were transfected into Hela cells as indicated. Lysates were prepared and IKK2-55 precipitated with an anti-Flag antibody and the precipitate analysed by Western blotting with an anti-Myc antibody (upper panel). The presence of Flag- IKK2-55 and myc-IKK2 in the cell extracts was confirmed by Western blotting (lower panels).

**Figure 2 (IKK2-55)**

Subcellular localization of IKK2-55. HUVEC were transfected with aYFP-IKK2-55 fusion construct together with CFP fusion genes for different cellular compartments and viewed under the fluorescence microscope. Left row shows localization of IKK2-55 in the yellow channel, the right row shows localization of the markers in the cyan channel. Upper panels: endoplasmatic reticulum (ER); middle panels: mitochondria (mito); lower panels: golgi.

**Figure 3 (IKK2-55)**

Influence of IKK2-55 on an NF-kB-dependent promoter. HUVEC were transfected with the 5xNF-kB-Luc construct together with different amounts of IKK2-55 as well as Ubiquitin-bgal as internal control and after 2 days stimulated with 100 U/ml TNF$\alpha$ for 6 hours. Luciferase values (relative light units) were normalized for b-gal expression. prom = promoter; unstim = unstimulated; stim = stimulated

**Figure 4 (IKK2-55)**

Effect of IKK2-55 (in combination with different kinases) on an NF-kB dependent promoter. HUVEC were transfected with the 5xNF-kB-Luc construct together with IKK2-55, with different kinases (IKK2, NIK, TAK1, p38) as indicated as well as Ubiquitin-bgal as internal control. In on case, cells were stimulated with 100 U/ml TNF$\alpha$ for 6 hours. Luciferase values are expressed as relative light units and were normalized for $\beta$-gal expression.

Dark columns: 500 ng IKK2-55; shaded columns: 0 ng IKK2-55

RLU-$\beta$gal: Relation Light Units per $\beta$-gal

**Figure 5 (LLR-J24)**

Phylogenetic tree including all known lectin-like genes of the human NK receptor complex:

The sequences of the CTLDs of the genes displayed were aligned and a phylogenetic tree constructed as described in the Examples. All sequences were chosen to start two amino acids before the first absolutely conserved cysteine in the domain. NKG2E and H are two alternatively spliced transcripts from the same gene. For LY49L a hypothetical sequence containing all three CTLD exons spliced comparably to the other genes were chosen, although only aberrantly spliced transcripts have been detected for this gene. The genes consistently found to be closer related to each other using different parameter settings for alignment and tree construction are indicated by brackets on the right. The scale below is a measure for substitution events. Preferential expression of the subfamilies in NK cells, T cells, endothelial cells (EC), monocytic cells (MO), dendritic cells (DC) or more broadly in leukocytes (LEUK) is indicated.

**Figure 6 and 7 (LLR-J24)**

Expression of recombinant LOX-1, LLR-J24 and CLEC-1 in HUVEC:

Esxpression plasmids for C-terminally FLAG-tagged LOX-1, LLR-J24 and CLEC-1 were transfected into HUVEC. Expression of the proteins was analyzed 24 h post-transfection following staining with anti-FLAG antibodies and secondary Texas Red-labeled antbodies.

Figure 6:

Comparison of the staining of non-permeabilized and permeabilized cells. In the left lanes formaldehyde-fixed non-permeabilized cells (fix) and in the right lanes permeabilized cells (fix/permeab) are displayed. The upper rows show staining with the anti-FLAG antibody, the lower rows staining of the DNA with Hoechst 333258 to visualize total cell number.

Figure 7:

The LLR-J24 construct without the stalk exon is indicated in Figure 2B ($\Delta$LLR-J24-1). Comparison of the staining of permeabilized cells for all four expression constructs at higher magnification.

**Figure 8 (LLR-J24)**

Downregulation of LLR-J24 mRNA during maturation of dendritic cells:

Figure 8A:

Northern blot of total RNA isolated from immature MoDC and cultures stimulated for 18 h with a combination of TNF-$\alpha$ and IL-1$\beta$. Hybridization with a LLR-J24 cDNA and a $\beta$-actin probe as control is shown.

Figure 8B:

Northern blot with RNA isolated from immature MoDC and HPDC, MoDC and HPDC stimulated with anti-CD40 antibodies or MoDC treated with a combination of anti-CD40 antibodies and zymosan A for 24 hours. The blot was consecutively hybridized with probes for LLR-J24, MDC and GAPDH as a loading control. The positions of 18s and 28s rRNA are indicated.

**Figures 9 and Figure 10 (LLR-J24)**

A fraction of human LLR-J24 mRNA contains the stalk exon sequences:

Figure 9:

RT-PCR of LLR-J24 mRNA. RNA from MoDC was reverse-transcribed using a specific LLR-J24 primer and PCR performed on the obtained cDNA using different primer pairs as indicated. (a) Displays the amplification of transcripts with and without the stalk exon for MoDC RNA, (b) displays the amplification of the 5'-part and (c) of the 3'-part of the stalk containing transcript for MoDC and HL60 RNA as set out under B in Figure 4A. Lane 6 displays the product obtained from a plasmid containing the full-length LLR-J24 cDNA.

Figure 10:

Schematic drawing of the oligonucleotide primers used for RT-PCR. The first primer pair (a) was designed to test the presence of the stalk exon fragment resulting in predicted 505 bp and 367 bp PCR products with and without the stalk exon fragment, respectively. The second primer pair (b) tested correct splicing of the 5'-part, the third primer pair (c) correct splicing of the 3'-part of the stalk exon containing transcripts.

c = cytoplasmic; TM = transmembering; CTLD1 = C-type lectin-like domain 1;
CTLD2 = C-type lectin-like domain 2; CTLD3 = C-type lectin-like domain 3

In the following Examples all temperatures are in degree Centigrade and are uncorrected.

Abbreviation listing:

| | |
|---|---|
| AA: | amino acid |
| BSA: | bovine serum albumin |
| CB: | cord blood: |
| CM: | culture medium |
| CTLD: | C-type lectin-like domain |
| DC:. | dendritic cell |
| dNTPs: | deoxynucleotide triphosphate |
| DTT: | dithiothreitol |
| ER: | endoplasmatic reticulum |
| LPS: | lipopolysaccharide |
| MHC: | major histocompatibility complex |
| MNC: | mononuclear cells |
| ORF: | open reading frame |
| PBS: | phosphate-buffered saline |
| PCR: | polymerase chain reaction |
| RT-PCR: | reverse-transcription PCR |
| SCF: | stem cell factor |

**Example 1: IKK2-55 gene**

cDNA clones comprising **SEQ ID NO: 1** of an IKK2-55 gene are isolated from libraries of PHA-activated lymphocytes mRNA and by sequence comparison using the BLAST algorithm with the GenBank databases.

In a yeast two hybrid screen using the C-terminal HLH domain of IKK2 as bait, the gene IKK2-55 was found. The initial clone comprising a C-terminal fragment is extended using ESTs and further sequencing of two IMAGE clones (#1: Clone ID=2048289; GenBank Accession No. = AI375687 and #2: Clone ID=2382861; GenBank Accession No. = AI759984). The nucleic and AA sequences are given in **SEQ ID NO:1** and **SEQ ID NO:2,** respectively. The first ATG at nucleotide position 58-60 is preceded by an in-frame stop codon at pos. 16-18. The ORF extends until a stop codon at pos. 1189-1191. IKK2-55 encodes a 377 AA protein that does not show any significant homology with entries in the databases. It also does not contain distinct protein motifs present in the Pfam and Prosite databases except a region of low compositional complexity at AA 45-58 and two coiled-coils at 116-259 and 287-323. A genomic clone exists in the database (AC013283.htgs) that resembles the human gene. Searching the human draft genomic sequence with the EST clones, e.g. AI375687, IKK2-55 localizes to chromosome 12q23.1. Thereby the IKK2-55 transcription start site localizes within 2 kb to the transcription start site of APAF1 (apoptotoc protease activating factor 1), implicating crosstalk in the regulation of these two genes.

**Example 2: IKK2-55 gene**

While the Y2HS provides a first information on the interaction between two proteins, these results has been confirmed by biochemical means. Expression vectors for IKK2 (with a Flag tag attached) and for IKK2-55 (with a myc tag attached) are transfected into HeLa cells. Two days later, extracts are prepared and IKK2 is precipitated with an anti Flag antibody. The precipitate is analyzed by Western blotting using anti myc antibody. The presence of a specific band in lane 3, **Figure 1,** indicates co-immunoprecipitation of IKK2-55 with IKK2. In the lower panel, whole cell extracts are probed for IKK2-55 and IKK2 using anti-Flag and anti-myc antibodies, respectively, to demonstrate that cells express the respective genes.

**Example 3: IKK2-55 gene**

Intracellular localization may provide evidence for the function of a protein. Transfection of a YFP-IKK2-55 fusion gene results in localization to perinuclear regions (**Figure 2**) that, by cotransfection of different specific markers appears to resemble the endoplasmatic reticulum (ER; the construct contains the targeting sequence of calreticulin fused to CFP; Clontech Living Colors ER Subcellular Localization Vector).

**Example 4: IKK2-55 gene (expression pattern)**

A MTN (multiple tissue Northern blot; Clontech) is hybridized with an IKK2-55 probe and two bands at 2.4 and 4.3 kb are detected predominantly in kidney, liver, placenta, spleen and heart.
In searching for ESTs using the TIGR human gene index, limited expression is found:

| Library name | Number of hits |
|---|---|
| Total fetus Nb2HF8 9w, Bento Soares and M. Fatima Bonaldo LC80 | 3 |
| Kidney, NCI-CGAP-Kid11 LE93 | 1 |
| Mixed germ cell tumors, NCI-CGAP-GC5 LD12 | 1 |
| Kidney, 2 pooled tumors (clear cell type), NCI-CGAP-Kidl2 LF2 | 1 |
| Pooled human melanocyte, fetal heart, and pregnant uterus, Soares NhHMPu S1 LA65 | 1 |

Virtual SAGE is performed (the string aaaataagcc is chosen by the program) and high expression in the human breast cancer line MDA-MB-468 that is PTEN -/- is detected. RT-PCR is performed using IKK2-55-specific primers and mRNA from stimulated and control EC. Enhanced IKK2-55 expression is found in EC that had been stimulated with IL-1, with LPS or with VEGF.

**Example 5: IKK2-55 gene**

Since IKK2-55 interacts with IKK2 as shown by genetic and biochemical means, IKK2-55 influence on NF-kB activity was tested. An NF-kB dependent promoter-luciferase reporter construct, namely 5xNF-kB-Luc, containing 5 copies of a NF-kB binding site, is used in transient transfections of HUVEC, with or without TNF$\alpha$ stimulation. The results are shown in **Figure 3** and demonstrate that, depending on the amounts used, IKK2-55 has either stimulatory (low, physiological amounts) or inhibitory (at high amounts) activity. When using the 5xNF-kB promoter, cotransfection of IKK2-55 together with kinases known to be involved in NF-kB signalling, such as IKK2, TAK1, NIK, p38, superinduce the expression of the reporter gene (**Figure 4**).

**Example 6: DINO gene (homology)**

cDNA clone comprising **SEQ ID NO:3** of the DINO gene isolated from libraries of skin microvascular endothelial cell mRNA and by sequence comparison using the BLASTX algorithm with the SWISSPROT Protein database is found to be homologous to the family of HERC proteins (domain homologous to E6 associated protein carboxy-terminus and RCC1 domain), especially to HERC3 (accession No. D25215). and Cep1 (accession no. AB027289).

The DINO gene of **SEQ ID NO:3** shows an overall homology to HERC3 and Cep1 of 37.8 % homology, but the homology is significantly higher (up to 70 %) in the defined protein domains. A characteristic difference exists between DINO and Cep1 in that Cep1 has an N-terminal extension of about 100 amino acids. The HECT domain of HERC proteins is involved in transfering ubiquitin to specific cellular target proteins destined for degradation or processing.

**Example 7: DINO gene (pathway profiling system)**

The potential of DINO to induce p53 transcriptional activity may be assayed in a signaling pathway profiling system in vivo. The system is based on cis-acting elements upstream from the luciferase reporter gene. When the pathway is activated by overexpression of DINO in transfectedHeLa or endothelial cells the expression of luciferase can be measured. In this way, the effects of different drugs, stimuli or co-transfected genes can be studied by assaying for luciferase expression.

**Example 8: LLR-J24 gene (homology)**

cDNA clones comprising **SEQ ID NO: 5** of an LLR-J24-1 gene are isolated from libraries of DC mRNA and detected in the GenBank expressed sequence tagged database (dbEST). Libraries are searched with short consensus sequences established from the NKG2-A, -C, -D and the human Ly49 genes (Houchins J.P. et al. J. Exp. Med. 173 [1991] 1017-1020; Bull C. et al. Genes and Immunity 1 [2000] 280-287). cDNA clones comprising **SEQ ID NO:** 7 of the splice variant LLR-J24-2 have been isolated by reverse transcription PCR from mRNA of monocyte-derived DCs. The cDNA clones comprising **SEQ ID NO: 5** and **SEQ ID NO: 7** were found by sequence comparison using the BLASTX algorithm to be homologous to the superfamily of CTLD receptors. To establish the relationship of LLR-J24 to the other known genes of the NKC the predicted amino acid sequences of the CTLDs are compared. Multiple alignment of the CTLD domains of all known lectin-like genes of the NK receptor complex is performed using the ClustalW method of the MacVector 6.5 software with the parameters: Open gap penalty - 7.0, extend gap penalty for pairwise alignment - 2.5, and for multiple alignment -1.0. Accession numbers for the molecules used in the alignment are: NKG2A (X54867), NKG2C (X54869), NKG2E (L14542), NKG2H (AF078550), NKG2D (X54870), LOX-1 (AF035776), CLEC-1 (AF200949), CLEC-2 (AF124841), DCIR (AJ133532), AICL (X96719), LLT-1 (AF133299), CD69 (L07555), KLRF1 (AF175206), MAFA (AF081675), and NKR-P1A (U11276). The hypothetical LY49L (hypLy49L) CTLD sequence was obtained by combining the corresponding 3 exons of the gene (AF213453), although a transcript with correctly spliced CRD exons according to the other CTLD genes has so far not been detected for LY49L. Phylogenetic trees are constructed by the MegAlign program of the DNASTAR software version 3.0 according to the nearest neighbor method.

Following multiple alignment using the ClustalW algorithm a phylogenetic tree is constructed (**Figure 5**). The data obtained display that LLR-J24 forms together with LOX-1, CLEC-1 and CLEC-2 a subfamily of closer related lectin-like receptors of the NKC. The highest similarity of LLR-J24 was seen with LOX-1 (44 %), CLEC-1 and CLEC-2 are somewhat more distantly related (37 %). The subfamily of CTLD NK receptors consisting of the NKG2 and the CD94 NK receptors are the next closest related proteins (between 28 % for NKG2-E and 33 % for NKG2-D). The AICL/LLT-1/CD69 group and the KLRF1, MAFA and NKR-P1 receptors are again somewhat more distant and are displayed in separate branches in a multiple alignment. The sequence of LLR-J24-1 displayed high similarity to the recently described murine DECTIN-1 cDNA with the exception that a stalk exon was missing from the human cDNA. It could be shown that a stalk exon is found spliced in a subfraction of about 5 to 10 % of the transcripts (**Figures 9 and 10**) as shown for the splice variant LLR-J24-2 in **SEQ ID NO:7.**

A comparison of the the corresponding predicted full-length protein sequences of human LLR-J24-2 and mouse DECTIN-1 show an identity of 59 % (similarity 69 %). The high similarity is conserved in the cytoplasmic and extracellular domains including the stalk domain. Close to the N-terminus of the cytoplasmic domain an ITAM-like sequence is present in LLR-J24 as well as the murine DECTIN-1. This sequence differs slightly from a classical ITAM motif as it is found in the CD3-$\gamma$ chain or the DAP12 molecule. The first tyrosine (residue 3) in the motif is 4 amino acid positions before the leucine (residue 7), whereas this distance is 3 amino acid positions in the classical $Yx_2Lx_{6-8}Yx_2L$ motif. The following residues of the motif are correctly placed. Both proteins further show consensus sites for N-glycosylation. The lectin-like domain of the human and murine versions lack most of the residues described to be involved in $Ca^{++}$-binding suggesting a non-carbohydrate ligand. The transmembrane domain does not display a charged amino acid indicating that association with ITAM-containing adapters such as the DAP molecules does not take place.

Thus from the similarity of LLR-J24 in the CTLD to the other lectin-like receptors and its expression profile (see below) it is to be expected that the LLR-J24 receptor has a proteineous ligand such as a lipoprotein, a MHC class I homologue or another transmembrane or secreted protein of cellular or parasite origin.

**Example 9: LLR-J24 gene**

Isolation and sequencing:

The complete cDNA of a gene of **SEQ ID NO:5** may be obtained by either of the following methods:

a) The method of Rapid Amplification of cDNA Ends (RACE) can be utilized to obtain the 5' end (see Frohman et al. Proc. Nat. Acad. Sci USA 85 [1988] 8998-9002. Briefly, specific oligonucleotides are annealed to mRNA and used to prime the synthesis of the cDNA strand. Following destruction of the mRNA with RNaseH, a poly C anchor sequence is added to the 3' end of the cDNA and the resulting fragment is amplified using a nested set of antisense primers and an anchor sequence primer. The amplified fragment is cloned into an appropriate vector and subjected to restriction and sequence analysis.

b) The polymerase chain reaction can be used to amplify the 5' end of the cDNA from human cDNA libraries using sequential rounds of nested PCR with two sets of primers. One set of antisense primers is specific to the 5' end of the partial cDNA and the other set of primers anneals to a vector specific sequence.

To obtain LLR-J24, RT-PCR was performed according to following protocol: First 40 pg of an oligonucleotide primer specific for LLR-J24 (5'- GATTCCATGTCTAGGGATCTAC - 3') is annealed with 3 $\mu$g of total RNA in a volume of 10 $\mu$l containing 50 mM Tris-HCL (pH 8.3), 75 mM KCl and 3 mM $MgCl_2$ by heating to 85° followed by cooling to 42° over a period of 30 to 60 min. First strand synthesis is then performed in 20 $\mu$l containing 50 mM Tris-HCL (pH 8.3), 75 mM KCl, 3 mM $MgCl_2$, 0.5 mM dNTPs, 20 mM DTT and 100 U Superscript Reverse Transcriptase (GIBCO/BRL, Rockville, MD). The cDNA contained in 1 $\mu$l of the first strand reaction mix is then employed as a template in a PCR reaction according to standard procedures. The following primers are used:

2nd-4th EXON: forward: 5'-GGGAATCCTATGCTTGGTAAT-3',
reverse: 5'-TGGAGATGGGTTTTCTTGGG-3';
5'-UT-STALK: forward: 5'-CTCCGGTAAGTACCTAGCCCA-3',
reverse: 5'-GTGGTTTTGACAGCTTTGGT-3';
STALK-3'-END: forward: 5'-GAGATCCAATTCAGGAAGCA-3',
reverse: 5'-TGGAGATGGGTTTTCTTGGG-3'.

The amplified products are cloned into an appropriate vector and subjected to restriction and sequence analysis. In this way **SEQ ID NO:5** and **SEQ ID NO:6** are obtained.

Cell culture, isolation of cells and RNA isolation for RT-PCR:

Cord blood progenitor cell derived DCs (CBDC): Cord blood (CB) samples were collected during healthy full-term deliveries at the obstetric department (Lainzer Hospital, Vienna, Austria) with the informed consent of the mothers. Mononuclear cells (MNC) are isolated within 10 hours of collection by Lymphoprep® (Nycomed Pharma AS, Oslo, Norway) density gradient centrifugation. $CD34^+$ cells are isolated from CB-MNC by positive immunomagetic cell sorting using the MACS CD34+ cell isolation kit from Miltenyi (Miltenyi Biotech, Bergisch Gladbach, Germany) according to the instructions of the manufacturer. The purity of isolated CD34+ cells ranges between 95 % and 99 %.

Immunomagnetically purified $CD34^+$ progenitor cells are seeded in 6-well plates (Costar, Cambridge, MA, USA) at an initial cell density of 5 x $10^4$ cells/ml and cultured in RPMI1640 medium (Life Technologies Ltd., Paisley, UK) supplemented with 10 % fetal calf serum (FCS), 2 mM glutamine and 50 $\mu$g/ml each of streptomycin and penicillin. This CM is further supplemented with SCF (50 U/ml) and Flt3-L (25 U/ml), both purchased from R&D Systems Inc., Minneapolis, MN, USA. On day 6, cell cultures are split to obtain a cell densitiy of 2 x $10^5$ cells/ml and fed with CM containing SCF (25 U/ml), Flt3-L (12.5 U/ml), GM-CSF (300 U/ml, Novartis, Basel, Switzerland), TNF$\alpha$ (50 U/ml; R&D) and TGF$\beta$ (5 ng/ml; R&D).

Monocyte derived DCs (MoDC):

Human monocytes obtained by countercurrent elutriation are seeded into Costar 6-well plates (Costar, Cambridge, MA, USA) at 8 x $10^5$ cells/ml in CM supplemented with GM-CSF (300 U/ml) and rIL-4 (200 U/ml). Cultures are fed on day 3 by exchanging half of the medium for fresh CM that had been supplemented with GM-CSF and IL-4 to achieve the final concentrations indicated above.

Stimulation of T cells, B cells, monocytes or DCs:

Human T cells, B cells and monocytes obtained by countercurrent elutriation T cells or B cells are cultured in CM and stimulated for 24 hours in the presence of PHA (10 μg/ml, Pharmacia Biotech, Uppsala, Sweden) or PWM (10 μg/ml, Sigma, St. Louis, MO, USA) and rhIL-4 (100 U/ml, Novartis Pharma, Basel, Switzerland), respectively. Monocytes, MoDC or HPDC are stimulated with 100 ng/ml LPS (Sigma) or in the presence of 4 μg/ml of anti-CD40 mAb (mAb clone 626.2) that is further cross-linked in solution by the addition of 2 μg/ml of F(ab')2 - fragments of goat-anti mouse IgG (Pierce Chemical Corp, Rockford, IL, USA) for 6 hours at 37°C. In some cases, zymosan A (25 μg/ml, Sigma) is used in conjunction with anti-CD40 mAb cross-linked by F(ab')2 - fragments of goat-anti mouse IgG. Alternatively, final maturation of MoDC is induced by incubation in the presence inflammatory cytokines such as TNFα(200 U/ml) and IL-1β (100 U/ml) for 18 to 24 hours as indicated in the Description of the Figures.

RNA isolation:

Total cellular RNA is isolated from the cells following cell lysis in TRIzol reagent (GIBCO/BRL, Rockville, MD, USA), extracting DNA and proteins with chloroform and precipitating the RNA by isopropanol.

**Example 10: LLR-J24 gene**

Expression in mammalian cells:

The receptors of a gene of **SEQ ID NO:5** and **SEQ ID NO:6** is expressed in either human embryonic kidney 293 (HEK293) cells or adherent CCL39 or dhfr CHO cells and the LLR-J24 gene of the invention is expressed in either human embryonic kidney 293 (HEK293) cells or adherent CCL39 or dhfr CHO cells or recombinant baculovirus-infected Sf9 cells. The expression vectors typically contain the coding regions free of 5' and 3' UTR's downstream of a strong promoter, e.g. CMV-IE and a Koscak sequence as well as an antibiotic resistance gene, e.g. neomycin or zeocin. The cells are transfected with individual receptor cDNAs by lipofectin and selected in the presence of 600 mg/ml G418 (neomycin). After 3 weeks of selection, individual clones are picked and expanded for further analysis. HEK293 or CHO cells transfected with the vector alone serve as negative controls. To isolate cell lines stably expressing the individual receptors, about 96 clones are typically selected and analyzed by RT PCR analysis. Receptor mRNAs are generally detectable in about 50 % of the G418-resistant clones analyzed. Recombinant baculoviruses for expression of the genes are generated, selected, purified and propagated using standard techniques.

To test for expression of LLR-J24 on the cell surface or in intracellular compartments, expression constructs were obtained in a modified pcDNA3.1/HisA vector (Invitrogen, Groningen, Netherlands) providing a cytomegalovirus enhancer/promoter and tested in transient transfection. The vector is modified by insertion of oligonucleotides containing a sequence encoding a Flag peptide. In the pcDNA3.1/N-Flag modified vector the Flag-encoding sequence is inserted between the HindIII and BamHI sites of the multiple cloning sites, in the pcDNA3.1/C-Flag modified vector between the XhoI and ApaI sites. The two differently modified vectors can be used to provide an N-terminal or C-terminal Flag epitope to proteins by cloning the respective cDNAs into corresponding restriction cleavage sites before or after the Flag sequence, respectively. The coding region of all three genes is then amplified from the cDNA by PCR using specific oligonucleotides providing EcoRI and NotI cleavage sites for insertion into the pcDNA3.1/N-Flag vector or EcoRI and XhoI cleavage sites for insertion into the pcDNA3.1/C-Flag vector. PCR, restriction enzyme digest, fragment purification and ligation into the vector is performed according to established procedures. All constructs were verified by complete sequencing of the inserted DNA fragment.

293 cells and HUVEC are seeded 24 hours prior to transfection in six-well tissue culture plates to reach 80-90 % confluency the next morning. 293 cells are transfected by the calcium phosphate method using the mammalian transfection kit (Stratagene, La Jolla, CA, USA). Transient transfections of HUVEC cells are carried out by using the Lipofectamine™ reagent (GIBCO/BRL, Rockville, MD).

Immunofluorescence assays are performed mainly as described in the following: 24 hours following transfection, cells transfected with the appropriate expression plasmids are washed twice with PBS, fixed for 10 min at room temperature with 3.7 % formaldehyde, 2 % sucrose in PBS and permeabilized for 5 min with 0.5 % Triton X-100 in PBS. Primary antibodies (anti-Flag M2 monoclonal antibody, Sigma, St. Louis, MO, USA) are diluted in PBS, 1 % BSA and incubated with the cells for 1 h at room temperature. Cells are washed 3 times for 5 minutes with PBS, incubated with Texas Red-labeled donkey anti-mouse IgG (Accurate Chemical, Westbury, NY, USA) for 1 hour at room temperature and washed with PBS again. To visualize cell nuclei a fluorescent groove-binding probe for DNA (Hoechst 333258, Sigma Chemicals, St. Louis, MO, USA) is added to the secondary antibody solution at 500 ng/ml. Results are analyzed in a Nikon Diaphot TMD flourescence microscope (Nikon Ltd.) connected to a cooled charge-coupled device camera (Kappa Gmbh, Gleichen, Germany).

Following recombinant expression LLR-J24-2 is found on the cell surface, whereas LLR-J24-1 accumulates intracellularly:

The type II lectin-like receptors have been shown to usually dimerize and to form either homodimers or heterodimers combining two different receptor chains. In certain cases the dimerization with a heterologous lectin-like receptor chain has been shown to be necessary for surface expression. This is, for example, the case for the NKG2/CD94 receptors. Therefore, the surface expression of FLAG-tagged recombinant LLR-J24 was evaluated in comparison to CLEC-1 and LOX-1 in 293 cells and HUVEC following transient transfection with the respective expression constructs. To evaluate surface expression staining of non-permeabilized cells with an anti-FLAG antibody are compared to parallel samples of cells which are permeabilized with Triton X-100 before the staining procedure. Constructs with LLR-J24-2 and LLR-J24-1 (with and without the stalk exon) were used. LOX-1 and full-length LLR-J24 efficiently accumulated at the cell surface of HLTVEC as shown by strong staining of non-permeabilized cells (**Figure 6**). In contrast, no detectable levels of the LLR-J24-1 isoform without stalk and the full-length CLEC-1 protein are visible on non-permeabilized cells, although significant intracellular staining of permeabilized cells is obtained. A closer analysis of permeabilized cells at higher magnification revealed that cells transfected with LOX-1 and LLR-J24-2 expression constructs display strong staining of perinuclear cellular compartments as well as surface expression visible over the whole cell (**Figure 7)**. The stalkless LLR-J24-1 appears to be homogenously distributed throughout the cytoplasm, but is not appearing at the surface. Similar results are obtained with 293 cells.

Thus these data show that the stalk sequence as given in **SEQ ID NO:10** is required for surface expression of LLR-J24, likely because it contains a signal for transport to the surface and may facilitates homodimer formation. These data exemplify that LLR-J24-2 functions as a surface receptor whereas LLR-J24-1 may possess an intracellular function.

**Example 11: LLR-J24 gene (production of recombinant cells and recombinant protein)**

Based on previous experience with the NK receptors and the experience in the production of recombinant lectin-like receptors, expression constructs may be prepared for the expression of the full-length transmembrane receptor as well as for soluble versions thereof. Stably transfected cells with recombinant expression of the full-length form are used for monoclonal antibody generation and for functional studies. Soluble receptors can be more easily obtained in purified form and larger quantity and will therefore be used for immunizations and for ligand identification and isolation. In the initial phase of the project, because no antibodies are available to monitor expression, constructs providing a tag to the N-terminal and C-terminal ends of the molecules may be used. For full-length expression constructs were prepared without and with FLAG-tags in the pCDNA vector (InVitrogen). These constructs are used for stably transfecting two different cell lines. Stably transfected cells may be employed for immunization of mice to obtain monoclonal antibodies. Secondly, a hybrid cell line between a mouse hybridoma and a human DC may be used. Such hybrid cells may not express endogenous LLR-J24 and therefore could be candidates for functional studies with stable transfectants because of its partial dendritic properties of human origin.

In addition constructs may be designed to prepare a soluble LLR-J24 receptor in larger quantity using the cDNA encoding the extracellular portion fused to a signal sequence for cellular export. For this purpose the baculovirus system as available from InVitrogen may be used. Based on published data such proteins are also competent to properly bind the HLA-E ligands of the NK receptors. Protein in mg amounts is produced as it is of advantage for immunizations and for experiments to isolate and expression clone potential ligands from cell types interacting with DCs such as T cells. Alternatively, immunoglobulin fusion proteins are produced in COS cells with the extracellular domain fused to the C-terminal end of the immunoglobulin part, since the receptor has type II orientation and may therefore need a free carboxyterminal end for proper binding.

**Example 12: LLR-J24 gene (generation of polyclonal and monoclonal antibodies)**

Since antibodies to the lectin-like receptors are sometimes difficult to produce, synthetic peptide from the C-terminal end of LLR-J24 are used (the last 15 amino acids), since this is a method to obtain a polyclonal rabbit antibody functional in Western blots which is helpful to monitor recombinant expression. These antibodies can also be affinity purified with the immunizing peptide and may be used to study cellular expression in FACS analysis. Furthermore, soluble recombinant receptors for immunization of rabbits and for the production of affinity-purified antibodies are used. This gives antibodies useful to determine surface expression of the receptor by flow cytometry and could also exert neutralizing activity on receptor -ligand interactions.

In addition, monoclonal antibodies may be obtained to finally obtain epitope-specifc reagents useful to study structure-function relationships of the receptor. Monoclonal antibodies are generated which mimic ligand binding as well as others which block receptor-ligand interactions. This goal can be achieved by using mouse cell lines stably transfected with the human gene. Expression in cells such as murine MT cells leads to proper surface expression and proper antigenic structure of the molecule, so that reagents with good binding properties to the protein on human dendritic are obtained. Combined immunization protocols using purified recombinant protein as well as recombinant cells are used.

**Example 13: LLR-J24 gene (characterization of potentially associated protein chains)**

In some instances lectin-like receptors have been shown to form disulfide-linked heterodimers with different lectin-like proteins and non-covalently associate with signaling adaptor molecules. For example, several isoforms of NKG2 proteins heterodimerize with the CD94 protein to form several variants of inhibitory and activating NK cell receptors. In addition, the triggering NKG2/CD94 receptors associate via their NKG2 part with the signaling adaptor DAP 12 (Lanier L.L. et al. Immunity 8 [1998] 693-701). DAP 12 contains ITAM motifs and functions to give activating signals to the cell. On the other hand, NKG2-A/CD94, the inhibitory form, contains ITIM motifs on the cytoplasmic tail of NKG2-A, which recruit and trigger inhibitory signals via SHP-1 and -2 phosphatases (Palmieri G. et al., J. Immunol. 162 [1999] 7181-8). Following surface labeling of recombinant cells and primary human DCs with biotin (EZ-Link biotinylation reagent, Pierce) LLR-J24 can be immunoprecipitated and potentially co-immunoprecipitating chains scored in Western blots of the precipitate using the corresponding avidin-HRP reagents. Surface-exposed protein chains co-immunoprecipitated and different in size from the LLR-J24 can thus be detected by this method and isolated.

To detect signaling adaptors, which are not sufficiently surface-exposed, metabolically label DCs with $S^{35}$ methionine can be used and scored for co-immunoprecipitating proteins following polyacrylamide gel separation of precipitated proteins. Furthermore, known proteins which could interact with the receptor such as the DAP molecules can be tested. Thus a potential interaction with activating and inhibitory signaling molecules is established.

**Example 14: LLR-J24 gene (ligand screening with LLR-J24 polynucleotide)**

A collection of putative receptor ligands has been assembled for screening. The collection comprises: various oxLDL compounds and derivatives, carbohydrate compounds, parasite membranes, soluble versions of MHC class I and homologous, MICA, MICB allelic forms, various UL16 binding proteins known to act via the related LOX-1 and NKG2 or other lectin-like receptors; naturally occurring compounds which may be putative agonists for a human lectin-like receptor, non-mammalian, biologically active peptides for which a mammalian counterpart has not yet been identified; and compounds not found in nature, but which activate lectin-like receptors with unknown natural ligands. This collection is used to initially screen the receptor for known ligands, using both functional (i.e. calcium and cAMP release, tyrosine and serine kinase activation, phosphatases such as SHP-1 and -2) as well as binding assays with the various labeled compounds.

The search for potential ligands is performed with recombinant cells and isolated soluble receptor. Primarily two potential sources of ligands can be investigated. Based on the homology and the close chromosomal localization of LLR-J24 in respect to LOX-1 and the NK receptors it is assumed that a carbohydrate ligand is unlikely, although a residual sugar-binding property has been found in some of the lectin-like receptors. In analogy to the NK receptors or LOX-1 a proteineous ligand or a lipoprotein is expected. Similar to LOX-1, oxidized LDL could be bound. As suggested by preliminary experiments with the related mouse Dectin-1 (Ariizumi K. et al. J. Biol. Chem. 275 [2000] 20157-20167), LLR-J24 is further expected to rather engage with surface molecules of T cells.

Activation of LLR-J24 expressing recombinant cells versus the LLR-J24 negative parental cell lines can be evaluated. Activation of $Ca^{++}$, NFκB and of several MAP kinase pathways is tested. Alternatively, LLR-J24 recombinant are combined with T cells and T cell membrane fractions to evaluate activation. On the one side it is conceivable that the property of binding to oxLDL also mediates binding of apoptotic cells and such a function would make sense in the context of DCs. Endocytosed apoptotic cell material would be again presented via MHC molecules to T cells and thus infection of cells as cause of apoptosis recognized. The direct binding to lipoproteins on parasites can be tested. Alternatively, LLR-J24 could be involved in the interaction with a surface protein on T cells, such as a MHC homologue, and this interaction might have accessory function for T cell activation. A soluble receptor is used to identify and isolate and clone the ligand. This is done by using expression libraries of T cell cDNA in retroviral vectors, which are available and can be used to express the cDNA library in cells to which the soluble receptor is not binding. Tagged soluble receptor can be used to isolate transduced cells expressing the proper ligand by FACS sorting and the cDNA can be recovered from the sorted cells. If the ligand would have a more complicated structure, e.g. if it would consist of two chains, and these could not be expressed in one cell using the described method, biochemical purification using affinity chromatography with the soluble receptor and standard protein separation techniques may be used.

**Example 15: LLR-J24 gene (ligand binding assays)**

Ligand binding assays provide a direct method for ascertaining receptor pharmacology and are adaptable to a high throughput format. The purified ligand for a receptor is radiolabeled to high specific activity (50-2000 Ci/mmol) for binding studies. A determination is then made that the process of radio labeling does not diminish the activity of the ligand towards its receptor. Assay conditions for buffers, ions, pH and other modulators such as nucleotides are optimized to establish a workable signal to noise ratio for both membrane and whole cell receptor sources. For these assays, specific receptor binding is defined as total associated radioactivity minus the radioactivity measured in the presence of an excess of unlabeled competing ligand. Where possible, more than one competing ligand is used to define residual nonspecific binding.

**Example 16: LLR-J24 gene (functional assays)**

The potential activation or inhibition of cellular signaling in the recombinant DC-like DC/Sp20 versus non-recombinant cells, which do not express LLR-J24 is evaluated. The second approach is to use preparations of monocyte-derived and cord blood-derived primary DCs and cultures matured by induction with CD40 ligand, which are active to stimulate MLR. In the first approach antibodies and potential soluble ligands such as oxidized LDL or apoptotic cell fragments for potential activation of intracellular signals in the recombinant cells versus the parental cells are scored. Increase in free $Ca^{++}$, induction of NFκB and the major MAP kinase pathways MEK/ERK, p38 and JNK are tested. Interference of antibodies with the triggering compound or release of inhibitory function of the receptor can be tested.

A potential contribution of the receptor to maturation of DCs and to T cell activation can be evaluated as follows. In assay 1, immature Langerhans-type DCs are prepared and the antibodies tested whether they would inhibit or promote DC maturation/activation during CD40 ligand stimulation. For this purpose the upregulation of CD80/CD86 is determined by flow cytometry. In assay 2 it is evaluated whether the antibodies and the soluble receptor prepared alters the capacity of DCs to stimulate allogeneic T cells. Immature as well as mature DCs are used as stimulators for allogeneic T cells following preincubation of the dendrite cells with the antibodies or of the T cells with soluble receptor. The preferred read-out of T cell stimulation is thymidine incorporation after 5 days. These experiments detect an influence of the receptor on DC maturation as well as on T cell activation. Thus an accessory function of LLR-J24 in the interaction with T cells is detected. Antibodies and soluble receptors which might block the potential interaction of LLR-J24 with ligands on T cells are important to modulate the immune response.

**Example 17: LLR-J24 gene (extract/cell supernatant screening)**

A large number of lectin-like receptors exist for which there remains, as yet, no cognate activating ligand (agonist). Thus, active ligands for these receptors may not be included within the ligands banks as identified to date. Accordingly, the CTLD receptor of the invention is also functionally screened (using calcium, cAMP, kinase functional screens) against tissue extracts to identify natural ligands. Extracts that produce positive functional responses can be sequencially subfractionated until an activating igand is isolated and identified.

**Example 18: LLR-J24 gene (calcium and cAMP functional assays)**

Lectin-like receptors which are expressed in HEK 293 cells have been shown to be coupled functionally to activation of PLC and calcium mobilization, cAMP stimuation and/or tyrosine and serine/threonine kinase activation or inhibition. Basal calcium levels in the HEK 293 cells in receptor-transfected or vector control cells were observed to be in the normal, 100 nM to 200 nM, range. HEK 293 cells expressing recombinant receptors are loaded with fura 2 and in a single day>150 selected ligands or tissue/cell extracts are evaluated for agonist induced calcium mobilization. Similarly, HEK 293 cells expressing recombinant receptors are evaluated for the stimulation or inhibition of cAMP production using standard cAMP quantitation assays. Agonists presenting a calcium transient or cAMP flucuation are tested in vector control cells to determine if the response is unique to the transfected cells expressing receptor.

**Example 19: LLR-J24 gene**

RNA regulation of a LLR-J24 gene of the invention:

LLR-J24 is preferentially expressed in DCs:

Since LOX-1, LLR-J241 and CLEC-1 are members of one subfamily, the expression of LLR-J24 and CLEC-1 mRNA in primary human DCs, monocytes, T and B lymphocytes as well as endothelial cells was investigated. The data obtained show that LLR-J24 mRNA is abundantly and selectively expressed in monocyte-derived DCs (MoDC) and in DCs derived from $CD34^+$ hematopoietic progenitor cells (HPDC). A small amount of LLR-J24 mRNA was also present in primary monocytes, whereas primary T and B lymphocytes as well as endothelial cells, treated or untreated with different stimuli, did not detectably express LLR-J24 transcripts. No expression was visible in Northern blots including several additional cell lines, i.e. Jurkat, RPMI8866, NKL and NK92. The size of the RNA in DCs was estimated to be 3.0 to 3.5 kb from the Northern blots, which corresponds to the expected transcript sizes assuming the use of the first or several consecutive polyadenylation signals detected in the gene in a region 1.6 to 1.9 kb downstream from the stop codon.

LLR-J24 expression is downregulated during functional maturation of DCs:

Activation and final maturation of DCs is associated with altered expression of genes that regulate their migration and function. It was therefore tested whether stimulation of DCs by inflammatory mediators, CD40-ligand or zymosan A would lead to a change in expression levels for LLR-J24 mRNA. As shown in **Figure 8A**, treatment of MoDC with a combination of IL-1$\beta$ and TNF-$\alpha$ for 18 to 24 hours resulted in a reproducible, three to five-fold reduction of LLR-J24 mRNA. Alternatively, HPDC are activated by mAb specific for the CD40 receptor in the absence or presence of zymosan A stimulating phagocytosis of the yeast particles (**Figure 8B).** Based on these results, it appears that downmodulation of LLR-J24 mRNA is especially pronounced in cultures treated with strong inducers of maturation such as TNF-$\alpha$ and IL-1$\beta$ or CD40 ligation combined with phagocytosis stimulation. Since MDC chemokine (CCL22) is known to be strongly induced in DCs upon their maturation, the same Northern blot was hybridised with a specific probe for MDC to confirm successful activation of MoDC or HPDC. As seen in **Figure 8**, a strong induction of MDC parallels downregulation of LLR-J24 transcripts in both types of DCs.

LLR-J24 transcripts are variably spliced:

Northern blot analysis was unable to distinguish transcripts which were predicted to differ by only 141 bases for LLR-J24-1 and -2. Therefore reverse transcription PCR is performed using a primer pair to amplify a fragment between the second and fourth exon. Two products of 505 bp and 367 bp are obtained corresponding in size to the predicted fragments with and without the stalk exon, respectively (**Figure 9 and Figure 10**). It is estimated that approximately 5 to 10 % of the products were of the size containing the stalk exon. This upper band is excised from the gel, subcloned and two of the obtained clones are sequenced. Both clones displayed correctly spliced stalk exons as depicted in **SEQ ID NO. 7** and **SEQ ID NO. 9.** The PCR fragments obtained were further analyzed with primer pairs which amplify fragments of the mRNA from the 5'-untranslated region to the stalk exon and from the stalk exon to the sixth exon, respectively (see **Figure 8B**). In both cases the major obtained products corresponded to the sizes predicted for the correctly spliced exons. These data show that a fraction of the LLR-J24 transcripts contained the stalk exon and was also correctly spliced at the other exons.

## SEQUENCE LISTING

### IKK2-55 gene: SEQ ID NO: 1

AATTCGGCACGAGGGTGAAGAAGCTGCCCTGGGCTTGTCGTCCTAGGGTCTCCAGAC**ATG**TCTGAGG
TGAAGAGCCGGAAGAAGTCGGGGCCCAAGGGAGCCCCTGCTGCGGAGCCCGGGAAGCGGAGCGAGGG
CGGGAAGACCCCCGTGGCCCGGAGCAGCGGAGGCGGGGGCTGGGCAGACCCCCGAACGTGCCTGAGC
CTGCTGTCGCTGGGGACGTGCCTGGGCCTGGCCTGGTTTGTATTTCAGCAGTCAGAAAAATTTGCAA
AGGTGGAAAACCAATACCAGTTACTGAAACTAGAAACCAATGAATTCCAACAACTTCAAAGTAAAAT
CAGTTTAATTTCAGAAAAGCTTGAGTCTACTGAAAGCATCCTGCAGGAAGCTACATCATCCATGTCT
TTGATGACCCAGTTTGAGCAGGAAGTATCCAACCTCCAAGATATCATGCATGACATTCAAAATAATG
AAGAGGTGCTCACTCAAAGGATGCAAAGCCTTAATGAGAAATTTCAGAATATTACGGATTTCTGGAA
GAGAAGCCTAGAAGAAATGAACATTAATACAGACATTTTCAAATCAGAAGCAAAACATATACATTCT
CAAGTAACTGTCCAAATTAACTCAGCTGAGCAGGAAATAAAATTGCTCACTGAACGGCTAAAAGATT
TGGAAGATAGCACACTAAGAAATATTAGAACAGTAAAAAGACAAGAAGAAGATCTCCTGCGAGT
AGAGGAGCAGCTAGGCTCTGATACAAAGGCAATTGAAAAGTTAGAAGAGGAACAGCATGCCCTCTTT
GCCAGAGATGAAGATCTGACTAATAAACTTTCCGACTACGAACCCAAAGTTGAAGAATGCAAGACAC
ATTTGCCAACAATTGAAAGTGCTATTCACTCTGTTCTCAGAGTCTCTCAGGATCTGATAGAAACAGA
AAAGAAAATGGAAGACTTGACTATGCAGATGTTTAATATGGAAGATGATATGCTGAAAGCAGTGTCT
GAAATAATGGAGATGCAGAAAACCCTTGAAGGAATTCAGTATGATAATAGCATATTAAAGATGCAAA
ATGAACTGGATATTCTAAAAGAAAAAGTTCATGATTTTATAGCATACTCAAGTACAGGAGAAAAGGG
AACTTTAAAAGAATATAATATAGAAAATAAAGGAATTGGTGGTGATTTT**TAA**ATTCATGACATTTAT
TCTGATGAACCATATCATTATACATAAATTGATTAGTCCATTGATTTTGAGTTACTTTGATACGCCT
CATCTTATCCTACATTATTGGAAAATAAATGAGATGTCCACACAAATGGATTATCCACATAATCAAA
GCTTATCTTTTTAAGCACTAAAACTTACTTCATTGTAACTATTTGAAATAGAAATAGTTCTTTAAAA
ATATTTCTCTACTTTTAAAACAAGATACTTAATATTTTTAATGTTTTTAAAGCATTAATGAACATC
ATCATTAATTTCACCTATTATATTGTACCCACATATATTGATGTCTCCAGGGGCTAATAAAGAGTAT
TTACCACTTCACTAAATGACCCCAATTTGCCATTTTCAAACCTATGTCTCATACCTTTTCTGTCACC
TGAACTTACTGTCAGGTTGGTTGGTAATGTGCCTGCCTCAGCCAGTTCACTTTCTTAAGTAATTCAC
TGCAGGTTAAGAATCCACACAACCAAGTTCATGGGAATTTTGTGTAAAATAAAAGGATAACATGCCT
GANGGAGAGCCTGGTGCCTTTGCTATGAATTAATATGCCCTTCNGAAACTTTAAAGCCTGGAAACAT
TTAGTTTTTTGTAAGGTTCTAANCTGTGTNACAGATACTGTAAGACTTTTAAAATCAGAGTAAGTAT
GTTCTTACATAAGGCCTTTCTTAGGAATTATGTGTATATAAATCTTTGAAATATCTTTATATACCTG
AGTTTTAGGTTCCAACTGTGTTAGACATGAGTTTGCTAGATCCATGGTCTCTAGATGGTTTACTTAA
ATAGGCCAGGCGCAGTGGCTCACACCTGTAATCCTAGCACTTTGGGAGGCCGAGGTGGGCAGATCAC
GAAGTCAGGAGATCAAGACCATCTGGCTAACAGGGTGAAACCCTGTCTCTACTAAAAAATACAAAAA
ATTAGCCAGGCGTGGTGGTGGGTGCCTGTAGTCCCAGCTACTCAGGAGGCTGAGGCAGGAGAATGGT

GTGAACCCGGGAGGCAGAGCTTGCAGTGAGCCGAGATCGCGCCACTGCACTCCAGCCTGAGTGGCAA
AGTGAGACTCTGTCTCAATAAATAAATAAATAAA
TAAAAATAAGCCTGTGGCCTGTTTTGAGGGAAAAGTAACATAACTCTGATAATTAATTATGATAATA
ACTATGATAGTTAAAAATACGTCATTTTAAAGATTTCCCAAACCACACCACTAAATTTGTTTTATGA
ATTGATTTTATTTTATTTGAAATTATATAATGTAGTCAACTGCAATATGGAAGTTTGTTCTGGTTTC
TTTGGTCAATAACCTTACCAGTTTTATAATGGCAGTTTTTGATGGAATTAAACACAAAGAACTTGAA
AGCAGTCCAGTTACAAAAAGGTAGTTGTGGTCAAGTAGTTTTAATATGTAAATTTGCAAGTTTTTGG
AGAACCCCCAAACTCCTTCTCCACTGGAGTTAGGAGGGCCTGTGGTAGACAGCGTGAATCATGAAAA
TAAGCCTGGAGAGTTCTGTTATTAAGGGGCATAGCATTAATTTTTGCAGTNTTAAAATTACACTTCT
TGACTTCTGAATGTCTCTTATTTGCGTGCACTGACAAAATTCTATTTGTTTACTTTTATATTAATGG
TAATAAAATAGATTATAAAGCAAAAAAAAAAAAAAAAAAAAAAAAA

**IKK2-55 polypeptide: SEQ ID NO: 2**

MSEVKSRKKSGPKGAPAAEPGKRSEGGKTPVARSSGGGGWADPRTCLSLLSLGTCLGLAWFVFQQSE
KFAKVENQYQLLKLETNEFQQLQSKISLISEKLESTESILQEATSSMSLMTQFEQEVSNLQDIMHDI
QNNEEVLTQRMQSLNEKFQNITDFWKRSLEEMNINTDIFKSEAKHIHSQVTVQINSAEQEIKLLTER
LKDLEDSTLRNIRTVKRQEEEDLLRVEEQLGSDTKAIEKLEEEQHALFARDEDLTNKLSDYEPKVEE
CKTHLPTIESAIHSVLRVSQDLIETEKKMEDLTMQMFNMEDDMLKAVSEIMEMQKTLEGIQYDNSIL
KMQNELDILKEKVHDFIAYSSTGEKGTLKEYNIENKGIGGDF*

**DINO nucleotide: SEQ ID NO:3**

AGCACTTGAAGTTCAGGCAGCGAGAGTTGACATGGGGCCAGGGCTGCGCCCCTGGGGCGGGTTGAAG
ACAGGGTGAGTCTCTTGATATTCAGGAAATCATCGCGCACCCAGTCACCAGCGTTCGGGAGCCTGTC
GCAGCGGGACCGACGGAATCCGGAGCAGGCGACAGGGCGCAGAAGCGGGATGTACTTCTGTTGGGGC
GCCGACTCCAGGGAGCTGCAGCGCCGGAGGACGGCGGGCAGCCCCGGGGCTGAGCTACTGCAGGCGG
CCAGCGGGGAGCGCCACTCTCTGCTGCTGCTGACCAACCACAGGGTCCTCTCGTGCGGAGACAACAG
CAGGGGTCAGCTGGGCCGCAGGGGCGCGCAGCGCGGGGAGCTGCCAGAACCAATTCAGGCATTGGAA
ACCCTAATTGTTGATCTCGTGAGCTGCGGGAAGGAGCACTCCCTGGCTGTGTGCCACAAAGGAAGGG
TCTTCGCATGGGGAGCTGGTTCTGAAGGGCAGCTGGGGATTGGAGAATTCAAGGAAATAAGTTTCAC
ACCTAAGAAAATAATGACTCTGAATGATATAAAAATAATACAAGTTTCCTGTGGACACTACCACTCC
CTGGCATTATCAAAAGATAGCCAAGTGTTTTCGTGGGGAAAGAACAGCCATGGGCAGCTGGGCTTGG
GGAAGGAGTTCCCCTCCCAAGCCAGCCCGCAGAGGGTGAGGTCCCTGGAGGGGATCCCACTGGCTCA
GGTGGCTGCCGGAGGGGCTCACAGCTTTGCCCTGTCTCTCTGTGGGACTTCGTTTGGCTGGGGAAGT
AACAGTGCCGGGCAGCTGGCCCTCAGTGGGCGTAATGTCCCAGTGCAAAGCAACAAGCCTCTCTCAG
TCGGTGCACTGAAGAATCTAGGTGTGGTTTATATCAGCTGTGGTGATGCACACACTGCGGTGCTTAC
CCAGGACGGGAAAGTGTTCACATTTGGAGACAATCGCTCTGGACAGCTGGGATACAGCCCCACTCCT

GAGAAGAGAGGTCCACAACTTGTGGAAAGAATTGATGGCCTAGTTTCGCAGATAGATTGTGGAAGTT
ATCACACCCTGGCATATGTGCACACCACTGGTCAGGTGGTATCTTTTGGTCATGGACCAAGTGACAC
AAGCAAGCCAACTCATC

CGGAGGCCCTGACAGAGAACTTTGACATTAGCTGCCTGATTTCTGCTGAAGACTTCGTGGATGTTCA
AGTCAAACACATTTTTGCTGGAACATATGCCAACTTTGTGACAACTCATCAGGATACTAGTTCCACA
CGTGCTCCCGGGAAAACCCTGCCAGAAATAAGCCGAATTAGCCAGTCCATGGCAGAAAAATGGATAG
CAGTGAAAAGAAGAAGTACTGAACATGAAATGGCTAAAAGTGAAATTAGAATGATATTTTCATCTCC
TGCTTGTCTGACTGCAAGTTTTTTAAAGAAAAGAGGAACTGGAGAAACGACTTCCATTGATGTGGAC
TTAGAAATGGCAAGAGATACCTTCAAGAAGTTAACAAAAAAGGAATGGATTTCTTCCATGATAACTA
CGTGTCTCGAGGATGATCTGCTCAGAGCTCTTCCATGCCATTCTCCACACCAAGAAGCTTTATCAGT
TTTCCTCCTGCTCCCAGAATGTCCTGTGATGCATGATTCTAAGAACTGGAAGAACCTGGTGGTTCCA
TTTGCAAAGGCTGTGTGTGAAATGAGTAAACAATCTTTGCAAGTCCTAAAGAAGTGTTGGGCATTTT
TGCAAGAATCTTCTCTGAATCCGCTGATCCAGATGCTTAAAGCAGCCATCATCTCTCAGCTGCTTCA
TCAGACTAAAACCGAACAGGATCACTGTAATGTTAAAGCTCTTTTAGGAATGATGAAAGAACTGCAT
AAGGTAAACAAAGCTAACTGTCGACTACCAGAAAATACTTTCAACATAAATGAACTCTCCAACTTAT
TAAACTTTTATATAGATAGAGGAAGACAGCTCTTTCGGGATAACCACCTGATACCTGCAGAAACCCC
CAGTCCTGTTATTTTCAGTGATTTTCCATTTATCTTTAATTCGCTATCCAAAATTAAATTATTGCAA
GCTGATTCACATATAAAGATGCAGATGTCAGAAAAGAAAGCATACATGCTTATGCATGAAACAATTC
TGCAAAAAAAGGATGAATTTCCTCCATCACCCAGATTTATACTTAGAGTCAGACGAAGTCGCCTGGT
TAAAGATGCTCTGCGTCAATTAAGTCAAGCTGAAGCTACTGACTTCTGCAAAGTATTAGTGGTTGAA
TTTATTAATGAAATTTGTCCTGAGTCTGGAGGGGTTAGTTCAGAGTTCTTCCACTGTATGTTTGAAG
AGATGACCAAGCCAGAATATGGAATGTTCATGTATCCTGAAATGGGTTCCTGCATGTGGTTTCCTGC
CAAGCCTAAACCTGAGAAGAAAAGATATTTCCTCTTTGGAATGCTGTGTGGACTCTCCTTATTCAAT
TTAAATGTTGCTAACCTTCCTTTCCCACTGGCTCTGTATAAAAAACTTCTGGACCAAAAGCCATCAT
TGGAAGATTTAAAAGAACTCAGTCCTCGGTTGGGGAAGAGTTTGCAAGAAGTTCTAGATGATGCTGC
TGATGACATTGGAGATGCGCTCTGCATACGCTTTTCTATACACTGGGACCAAAATGATGTTGACTTA
ATTCCAAATGGGATCTCCATACCTGTGGACCAAACCAACAAGAGAGACTATGTTTCTAAGTATATTG
ATTACATTTTCAACGTCTCTGTAAAAGCAGTTTATGAGGAATTTCAGAGAGGATTTTATAGAGTCTG
TGAGAAGGAGATACTTAGACATTTCTACCCTGAAGAACTAATGACAGGAATCATTGGAAATACTGAT
TATGACTGGAAACAGTTTGAACAGAATTCAAAGTATGAGCAAGGATACCAAAAATCACATCCTACTA
TACAGTTGTTTTGGAAGGCTTTCCACAAACTAACCTTGGATGAAAAGAAAAAATTCCTCTTTTTCCT
TACAGGACGTGATAGGCTGCATGCAAGAGGCATACAGAAAATGGAAATAGTATTTCGCTGTCCTGAA
ACTTTCAGTGAAAGAGATCACCCAACATCAATAACTTGTCATAATATTCTCTCCCTCCCTAAGTATT
CTACAATGGAAAGAATGGAGGAAGCACTTCAAGTAGCCATCAACAACAACAGAGGATTTGTCTCACC
CATGCTCACACAGTCATAATCACCTCTGAGAGACTCAGGGTGGGCTTTCTCGCACTTGGATCCTTCT
GTTCTTCCTTACACCTAAATAATACAAGAGATTAATGAATAGTGGTTAGAAGTAGTTGAGGGAGAGA

TTGGGGGAATGGGGAGATGATGATGATGGTCAAAGGGTGCAAAATCTCACACAAGACTGAGGCAGGA
GAATAGGGTACAGAGATAGGGATCTAAGGATGACTTGGACACACTCCCTGGCACTGAAGAGTCTGAA
CACTGGCCTGTGATTGGTCCATTCCAGGACCTTCATTTGCATAAGGTATCAAACCACATCAGCCTCT
GATTGGCCATGGGCCAGACCTGCACTCTGGCCAATGATTGGTTCATTCCAGGACATTCATTTGCATA
AGGAGTCAAACCACACCAGTCTTGGATTGGCTGTGAG
CCAATTCACCTCAGTCTCTAATTGGCTGTGAGTCAGTCTTTCATTTACATAGGGTGTAACCATCAAG
AAACCTCTACAGGGTACTTAAGCCCCAGAAGATTTTGCTACCAGGGCTCTTGAGCCACTTGCTCTAG
CCCACTCCCACCCTGTGGAATGTACTTTCACTTTTGCTGCTTCACTGCCTTGTGCTCCAATAAATCC
ACTCCTTCACCACCCAAA

**DINO polypeptide: SEQ ID NO:4**

MYFCWGADSRELQRRRTAGSPGAELLQAASGERHSLLLLTNHRVLSCGDNSRGQLGRRGAQRGELPE
PIQALETLIVDLVSCGKEHSLAVCHKGRVFAWGAGSEGQLGIGEFKEISFTPKKIMTLNDIKIIQVS
CGHYHSLALSKDSQVFSWGKNSHGQLGLGKEFPSQASPQRVRSLEGIPLAQVAAGGAHSFALSLCGT
SFGWGSNSAGQLALSGRNVPVQSNKPLSVGALKNLGVVYISCGDAHTAVLTQDGKVFTFGDNRSGQL
GYSPTPEKRGPQLVERIDGLVSQIDCGSYHTLAYVHTTGQVVSFGHGPSDTSKPTHPEALTENFDIS
CLISAEDFVDVQVKHIFAGTYANFVTTHQDTSSTRAPGKTLPEISRISQSMAEKWIAVKRRSTEHEM
AKSEIRMIFSSPACLTASFLKKRGTGETTSIDVDLEMARDTFKKLTKKEWISSMITTCLEDDLLRAL
PCHSPHQEALSVFLLLPECPVMHDSKNWKNLVVPFAKAVCEMSKQSLQVLKKCWAFLQESSLNPLIQ
MLKAAIISQLLHQTKTEQDHCNVKALLGMMKELHKVNKANCRLPENTFNINELSNLLNFYIDRGRQL
FRDNHLIPAETPSPVIFSDFPFIFNSLSKIKLLQADSHIKMQMSEKKAYMLMHETILQKKDEFPPSP
RFILRVRRSRLVKDALRQLSQAEATDFCKVLVVEFINEICPESGGVSSEFFHCMFEEMTKPEYGMFM
YPEMGSCMWFPAKPKPEKKRYFLFGMLCGLSLFNLNVANLPFPLALYKKLLDQKPSLEDLKELSPRL
GKSLQEVLDDAADDIGDALCIRFSIHWDQNDVDLIPNGISIPVDQTNKRDYVSKYIDYIFNVSVKAV
YEEFQRGFYRVCEKEILRHFYPEELMTGIIGNTDYDWKQFEQNSKYEQGYQKSHPTIQLFWKAFHKL
TLDEKKKFLFFLTGRDRLHARGIQKMEIVFRCPETFSERDHPTSITCHNILSLPKYSTMERMEEALQ
VAINNNRGFVSPMLTQS*

**LLR-J24-1 nucleotide: SEQ ID NO:5**

1 GGTTGAACTA CTTAAGCTTA ATTTGTTAAA CTCCGGTAAG TACCTAGCCC ACATGATTTG
61 ACTCAGAGAT TCTCTTTTGT CCACAGACAG TCATCTCAGG AGCAGAAAGA AAAGAGCTCC
121 CAAATGCTAT ATCTATTCAG GGGCTCTCAA GAACAATGGA ATATCATCCT GATTTAGAAA
181 ATTTGGATGA AGATGGATAT ACTCAATTAC ACTTCGACTC TCAAAGCAAT ACCAGGATAG
241 CTGTTGTTTC AGAGAAAGGA TCGTGTGCTG CATCTCCTCC TTGGCGCCTC ATTGCTGTAA
301 TTTTGGGAAT CCTATGCTTG GTAATACTGG TGATAGCTGT GGTCCTGGGT ACCATGGGGG
361 TTCTTTCCAG CCCTTGTCCT CCTAATTGGA TTATATATGA GAAGAGCTGT TATCTATTCA
421 GCATGTCACT AAATTCCTGG GATGGAAGTA AAAGACAATG CTGGCAACTG GGCTCTAATC

```
481  TCCTAAAGAT AGACAGCTCA AATGAATTGG GATTTATAGT AAAACAAGTG TCTTCCCAAC
541  CTGATAATTC ATTTTGGATA GGCCTTTCTC GGCCCCAGAC TGAGGTACCA TGGCTCTGGG
601  AGGATGGATC AACATTCTCT TCTAACTTAT TTCAGATCAG AACCACAGCT ACCCAAGAAA
661  ACCCATCTCC AAATTGTGTA TGGATTCACG TGTCAGTCAT TTATGACCAA CTGTGTAGTG
721  TGCCCTCATA TAGTATTTGT GAGAAGAAGT TTTCAATGTA AGAGGAAGGG TGGAGAAGGA
781  GAGAGAAATA TGTGAGGTAG TAAGGAGGAC AGAAAACAGA ACAGAAAAGA GTAACAGCTG

841  AGGTCAAGAT AAATGCAGAA AATGTTTAGA GAGCTTGGCC AACTGTAATC TTAACCAAGA
901  AATTGAAGGG AGAGGCTGTG ATTTCTGTAT TTGTCGACCT ACAGGTAGGC TAGTATTATT
961  TTTCTAGTTA GTAGATCCCT AGACATGGAA TCTACTAACT AAAAAAAAAA AAAAA
```

## LLR-J24-1 poylypeptide: SEQ ID NO:6

```
  1    MEYHPDLENL DEDGYTQLHF DSQSNTRIAV VSEKGSCAAS PPWRLIAVIL GILCLVILVI
 61    AVVLGTMGVL SSPCPPNWII YEKSCYLFSM SLNSWDGSKR QCWQLGSNLL KIDSSNELGF
121    IVKQVSSQPD NSFWIGLSRP QTEVPWLWED GSTFSSNLFQ IRTTATQENP SPNCVWIHVS
181    VIYDQLCSVP SYSICEKKFS M
```

## LLR-J24-2 nucleotide: SEQ ID NO:7

```
   1 GGTTGAACTA CTTAAGCTTA ATTTGTTAAA CTCCGGTAAG TACCTAGCCC ACATGATTTG
  61 ACTCAGAGAT TCTCTTTTGT CCACAGACAG TCATCTCAGG AGCAGAAAGA AAAGAGCTCC
 121 CAAATGCTAT ATCTATTCAG GGGCTCTCAA GAACAATGGA ATATCATCCT GATTTAGAAA
 181 ATTTGGATGA AGATGGATAT ACTCAATTAC ACTTCGACTC TCAAAGCAAT ACCAGGATAG
 241 CTGTTGTTTC AGAGAAAGGA TCGTGTGCTG CATCTCCTCC TTGGCGCCTC ATTGCTGTAA
 301 TTTTGGGAAT CCTATGCTTG GTAATACTGG TGATAGCTGT GGTCCTGGGT ACCATGGCTA
 361 TTTGGAGATC CAATTCAGGA AGCAACACAT TGGAGAATGG CTACTTTCTA TCAAGAAATA
 421 AAGAGAACCA CAGTCAACCC ACACAATCAT CTTTAGAAGA CAGTGTGACT CCTACCAAAG
 481 CTGTCAAAAC CACAGGGGTT CTTTCCAGCC CTTGTCCTCC TAATTGGATT ATATATGAGA
 541 AGAGCTGTTA TCTATTCAGC ATGTCACTAA ATTCCTGGGA TGGAAGTAAA AGACAATGCT
 601 GGCAACTGGG CTCTAATCTC CTAAAGATAG ACAGCTCAAA TGAATTGGGA TTTATAGTAA
 661 AACAAGTGTC TTCCCAACCT GATAATTCAT TTTGGATAGG CCTTTCTCGG CCCCAGACTG
 721 AGGTACCATG GCTCTGGGAG GATGGATCAA CATTCTCTTC TAACTTATTT CAGATCAGAA
 781 CCACAGCTAC CCAAGAAAAC CCATCTCCAA ATTGTGTATG GATTCACGTG TCAGTCATTT
 841 ATGACCAACT GTGTAGTGTG CCCTCATATA GTATTTGTGA GAAGAAGTTT TCAATGTAAG
 901 AGGAAGGGTG GAGAAGGAGA GAGAAATATG TGAGGTAGTA AGGAGGACAG AAAACAGAAC
 961 AGAAAAGAGT AACAGCTGAG GTCAAGATAA ATGCAGAAAA TGTTTAGAGA GCTTGGCCAA
1021 CTGTAATCTT AACCAAGAAA TTGAAGGGAG AGGCTGTGAT TTCTGTATTT GTCGACCTAC
1081 AGGTAGGCTA GTATTATTTT TCTAGTTAGT AGATCCCTAG ACATGGAATC TACTAACTAA
1141 AAAAAAAAAA AAA
```

**LLR-J24-2 polypeptide: SEQ ID NO:8**

```
  1 MEYHPDLENL DEDGYTQLHF DSQSNTRIAV VSEKGSCAAS PPWRLIAVIL GILCLVILVI
 61 AVVLGTMAIW RSNSGSNTLE NGYFLSRNKE NHSQPTQSSL EDSVTPTKAV KTTGVLSSPC
121 PPNWIIYEKS CYLFSMSLNS WDGSKRQCWQ LGSNLLKIDS SNELGFIVKQ VSSQPDNSFW
181 IGLSRPQTEV PWLWEDGSTF SSNLFQIRTT ATQENPSPNC VWIHVSVIYD QLCSVPSYSI
241 CEKKFSM
```

**LLR-J24-stalk nucleotide: SEQ ID NO:9**

```
  1        CTA TTTGGAGATC CAATTCAGGA AGCAACACAT TGGAGAATGG CTACTTTCTA
 54 TCAAGAAATA AAGAGAACCA CAGTCAACCC ACACAATCAT CTTTAGAAGA CAGTGTGACT
114 CCTACCAAAG CTGTCAAAAC CACAG
```

**LLR-J24-stalk peptide: SEQ ID NO:10**

```
1 AIW RSNSGSNTLE NGYFLSRNKE NHSQPTQSSL EDSVTPTKAV KTT
```

## Claims

1. An isolated DINO gene comprising a nucleotide sequence encoding a DINO polypeptide of SEQ ID NO:4.

2. An isolated DINO gene comprising the nucleotide sequence of SEQ ID NO:3.

3. An isolated DINO polypeptide of SEQ ID NO:4.

4. A vector comprising a gene according to any one of claims 1 or 2.

5. An expression system comprising an DNA or RNA molecule, wherein said expression system or part thereof is capable of producing a polypeptide according to claim 3, when said expression system or part thereof is present in a compatible host cell.

6. A recombinant host cell produced by transforming or transfecting a host cell with the expression system according to claim 5, such, that the host cell, under appropriate culture conditions, produces a polypeptide according to claim 3.

7. An isolated host cell comprising an expression system according to claim 6.

8. A diagnostic kit for a disease or susceptibility to a disease, comprising as a main component

   a) a gene according to any one of claims 1 or 2,
   b) a nucleotide sequence complementary to that of (a),
   c) a polypeptide according to claim 3, or
   d) an antibody to a polypeptide according to claim 3.

9. An antibody against a polypeptide according to claim 3.

10. An immunological/vaccine formulation (composition) which, when introduced into a mammalian host, induces an immunological response in that mammal to a polypeptide of claim 3, wherein the composition comprises a polypeptide according to claim 3, or a gene according to any one of claims 1 or 2.

**11.** A screening assay for identifying an agonist or an antagonist of a polypeptide according to claim 3, which assay comprises as a main component

a) a polypeptide according to claim 3,
b) a recombinant cell expressing a polypeptide according to claim 3,
c) a cell membrane expressing a polypeptide according to claim 3, or
d) an antibody to a polypeptide according to claim 3.

**12.** A method of identfying an agonist or antagonist of a polypeptide according to claim 3, which comprises

A) contacting

a) a polypeptide according to any claim 3,
b) a recombinant cell expressing a polypeptide according to claim 3,
c) a cell membrane expressing a polypeptide according to claim 3, or
d) an antibody to a polypeptide according to claim 3,

with a candidate compound,
B) determining the effect of the candidate compound on any of a), b), c) or d);
C) choosing an agonist or antagonist determined in step B).

**13.** A method of treating abnormal conditions related to both an excess of and insufficient level of expression of a gene according to any one of claims 1 or 2; or related to both an excess of and insufficient activity of a polypeptide according to claim 3, comprising administering a therapeutically amount of a soluble form of a polypeptide according to claim 3 to a subject in need of said treating.

Figure 1 / 10

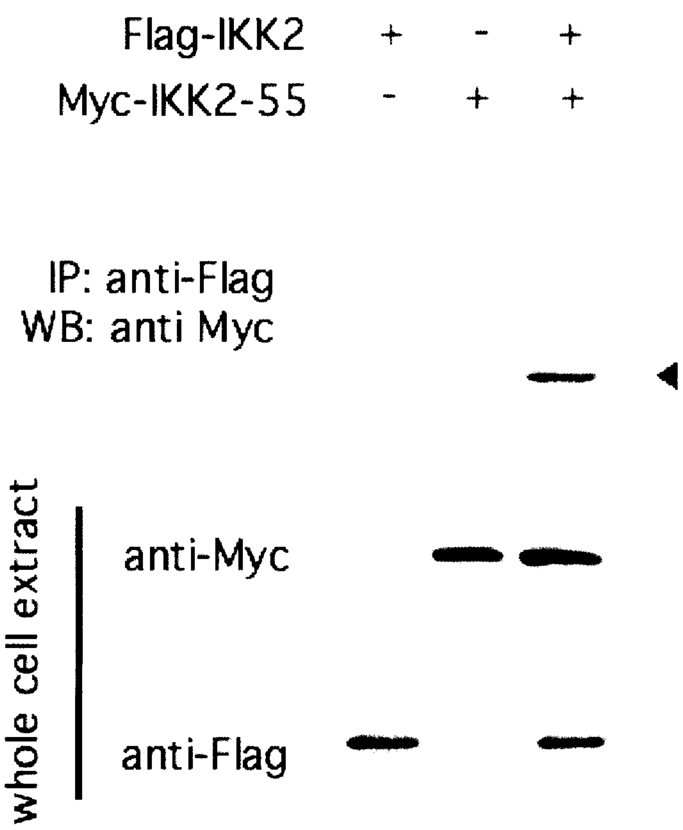
Flag-IKK2 + - +
Myc-IKK2-55 - + +
IP: anti-Flag
WB: anti Myc
whole cell extract
anti-Myc
anti-Flag

Figure 2 / 10

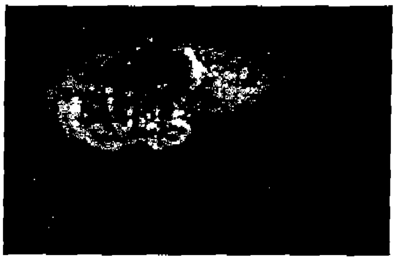

YFP-IKK2-55

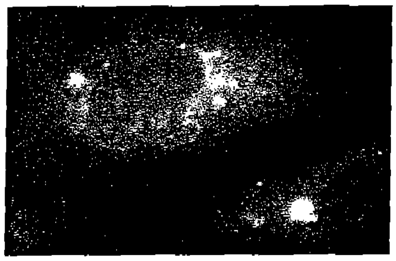

CFP-ER

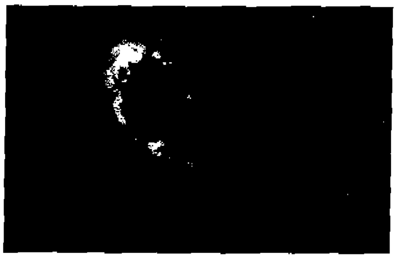

YFP-IKK2-55

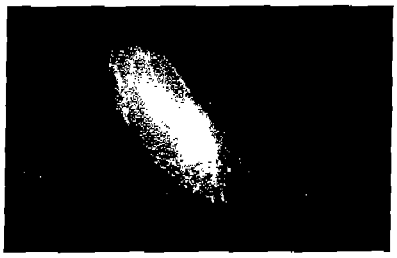

CFP-mito

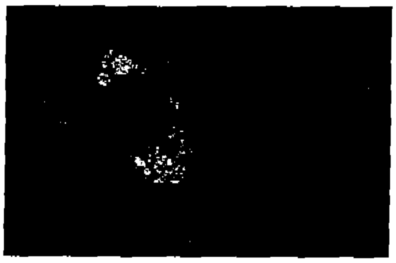

YFP-IKK2-55

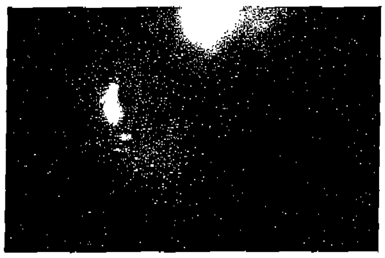

CFP-golgi

Figure 3 / 10

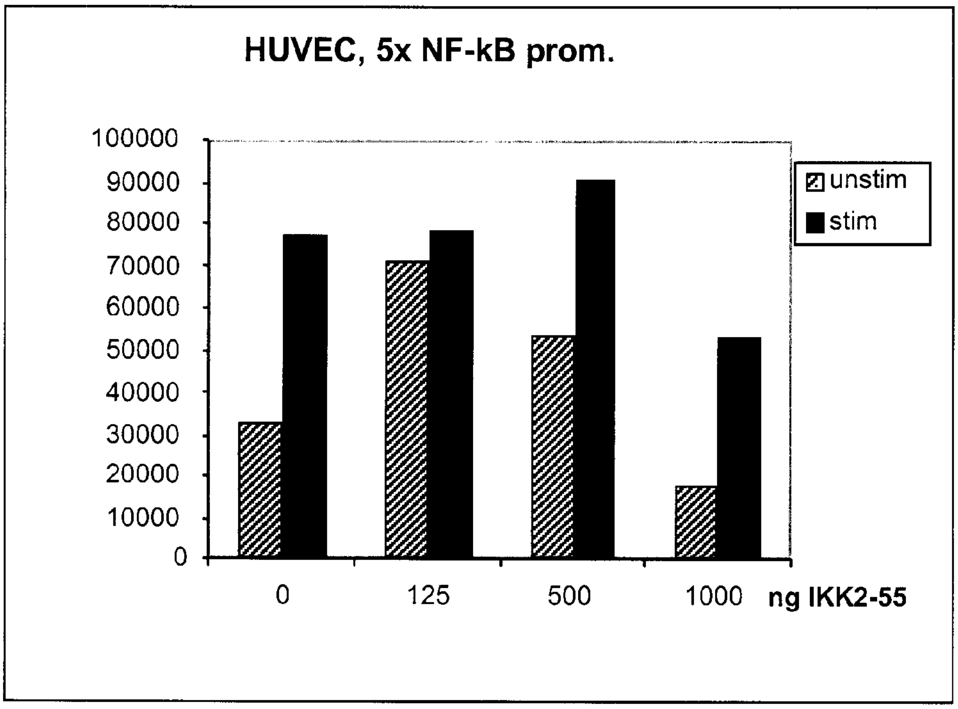
HUVEC, 5x NF-kB prom.
100000
90000
80000
70000
60000
50000
40000
30000
20000
10000
0
unstim
stim
0
125
500
1000
ng IKK2-55

Figure 4 / 10

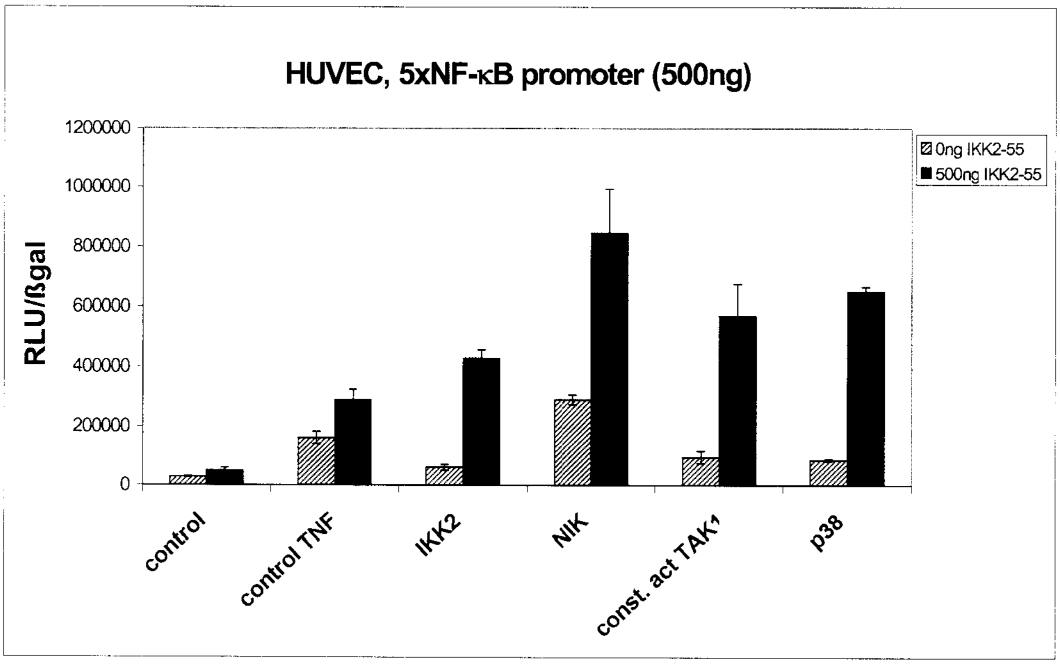
HUVEC, 5xNF-κB promoter (500ng)
RLU/ßgal
1200000
1000000
800000
600000
400000
200000
0
0ng IKK2-55
500ng IKK2-55
control
control TNF
IKK2
NIK
const. act TAK1
p38

Figure 5 / 10

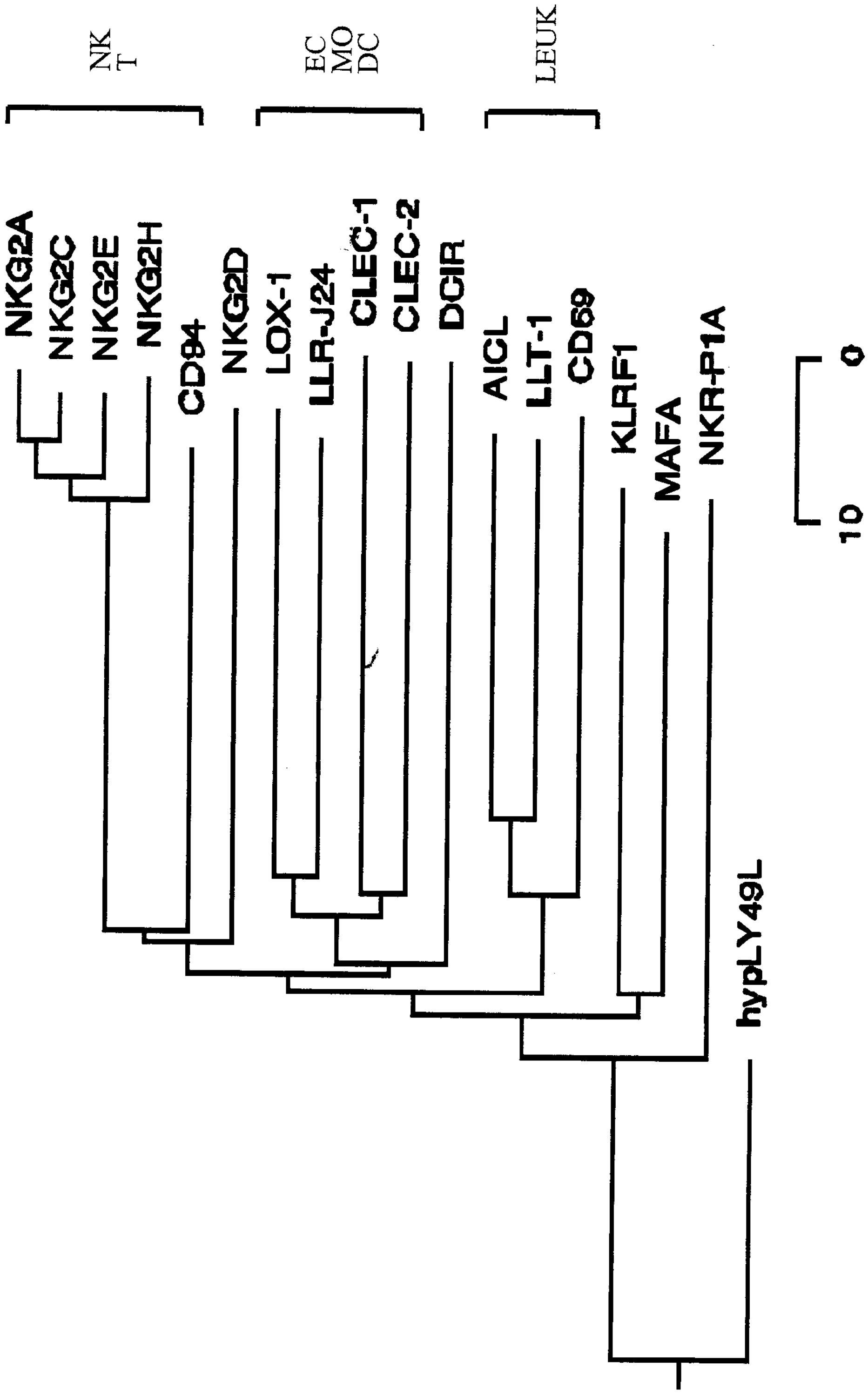
NK
T
EC
MO
DC
LEUK
NKG2A
NKG2C
NKG2E
NKG2H
CD94
NKG2D
LOX-1
LLR-J24
CLEC-1
CLEC-2
DCIR
AICL
LLT-1
CD69
KLRF1
MAFA
NKR-P1A
hypLY49L
0
10

Figure 6 / 10

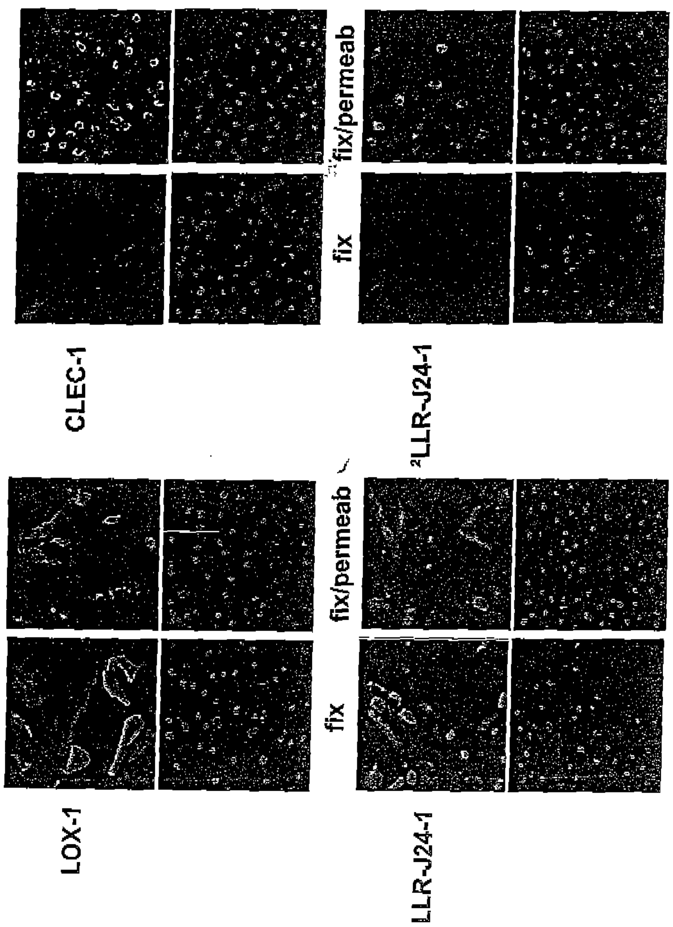

Figure 7 / 10

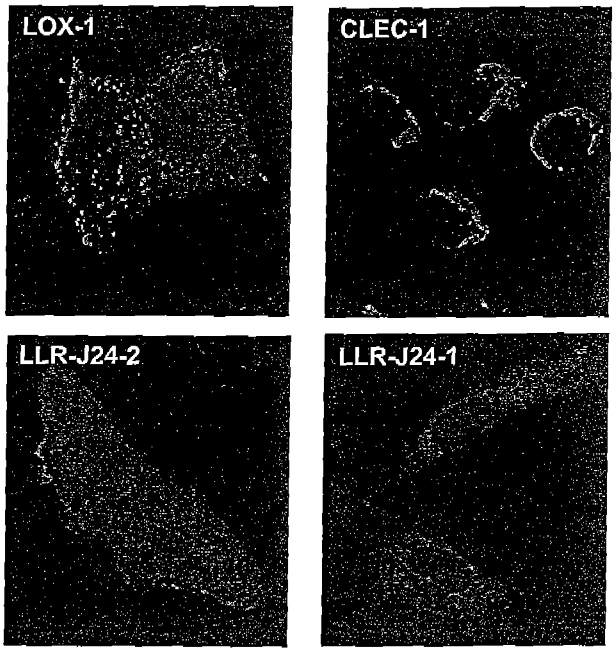

Figure 8 / 10

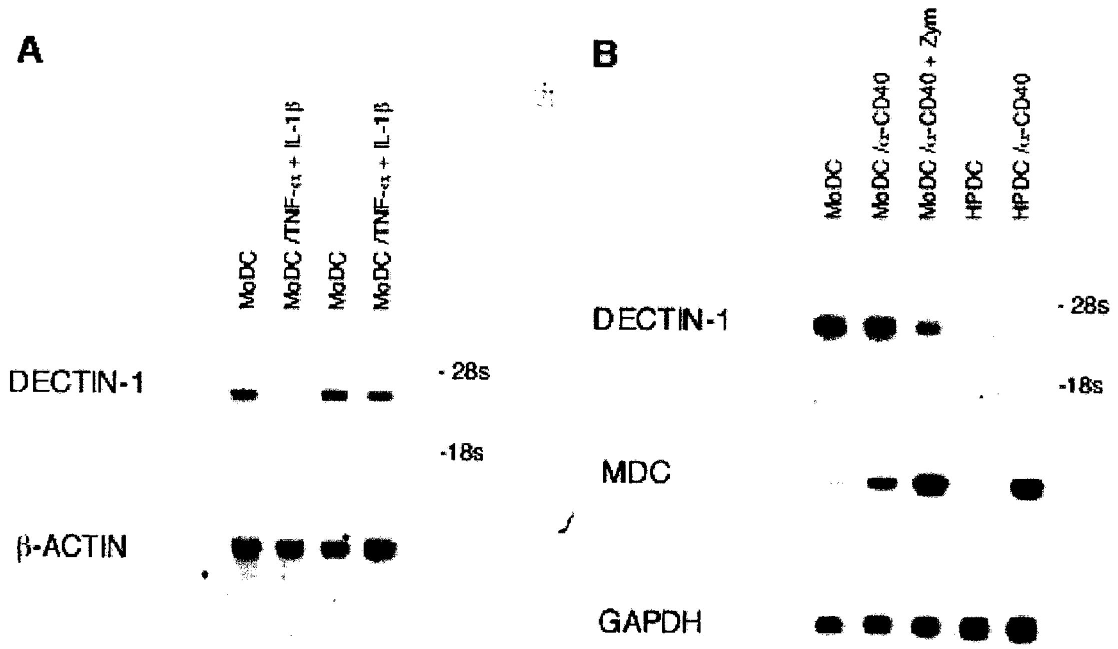
A
MoDC
MoDC /TNF-α + IL-1β
MoDC
MoDC /TNF-α + IL-1β
DECTIN-1
- 28s
-18s
β-ACTIN
B
MoDC
MoDC /α-CD40
MoDC /α-CD40 + Zym
HPDC
HPDC /α-CD40
DECTIN-1
- 28s
-18s
MDC
GAPDH

Figure 9 / 10

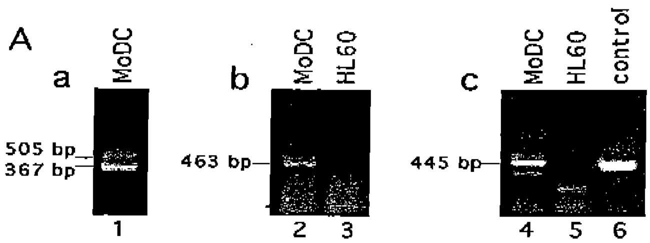
A
a
MoDC
505 bp
367 bp
1
b
MoDC
HL60
463 bp
2
3
c
MoDC
HL60
control
445 bp
4
5
6

Figure 10 / 10

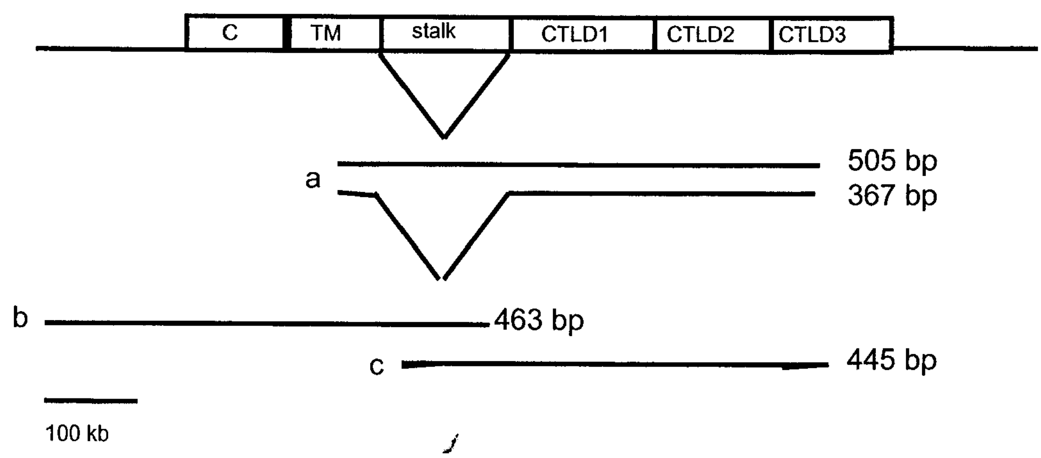
C
TM
stalk
CTLD1
CTLD2
CTLD3
a
505 bp
367 bp
b
463 bp
c
445 bp
100 kb

European Patent Office

# PARTIAL EUROPEAN SEARCH REPORT

which under Rule 63 of the European Patent Convention shall be considered, for the purposes of subsequent proceedings, as the European search report

Application Number
EP 07 10 5231

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CRUZ C ET AL: "HERC3 binding to and regulation by ubiquitin" FEBS LETTERS, ELSEVIER, AMSTERDAM, NL, vol. 488, no. 1-2, 12 January 2001 (2001-01-12), pages 74-80, XP004341426 ISSN: 0014-5793 * the whole document * ----- | 1-13 | INV. C12N9/00 |
| X | SCHWARZ S E ET AL: "Characterization of Human HECT Domain Family Members and Their Interaction with UbcH5 and UbcH7" JOURNAL OF BIOLOGICAL CHEMISTRY, AMERICAN SOCIETY OF BIOLOCHEMICAL BIOLOGISTS, BIRMINGHAM,, US, vol. 273, no. 20, May 1998 (1998-05), pages 12148-12154, XP002190154 ISSN: 0021-9258 * the whole document * ----- | 1-13 | |
| | -/-- | | TECHNICAL FIELDS SEARCHED (IPC) |
| | | | C12N |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

4

| Place of search | Date of completion of the search | Examiner |
|---|---|---|
| Munich | 10 December 2007 | Mueller, Frank |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04E07)

European Patent Office

# PARTIAL EUROPEAN SEARCH REPORT

Application Number
EP 07 10 5231

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| X | JI Y. ET AL.,: "structure of highly conserved herc2 gene and of multiple partially duplicated paralogs in human" GENOME RESEARCH, vol. 10, 2000, pages 319-329, XP002461405 * the whole document * ----- | 1-13 | |
| | | | TECHNICAL FIELDS SEARCHED (IPC) |

4

EPO FORM 1503 03.82 (P04C10)

European Patent Office

**INCOMPLETE SEARCH SHEET C**

Application Number
EP 07 10 5231

Although claim 13 is directed to a method of treatment of the human/animal body (Article 53(c) EPC), the search has been carried out and based on the alleged effects of the compound/composition.

-----

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4946778 A


### Non-patent literature cited in the description


- **DE MARTIN et al.** *Arterioscler. Thromb. Vasc. Biol.,* 2000, vol. 20, e83-e88
- **WRIGHTON et al.** *J. Exp. Med.,* 1996, vol. 183, 1013-1022
- **OITZINGER et al.** *Blood,* 2001, vol. 97, 1611-1617
- **HOFER-WARBINEK et al.** *J. Biol. Chem.,* 2000, vol. 275, 22064-22068
- **CINES et al.** *Blood,* 1998, vol. 91, 3527-3561
- **MANCONI et al.** *Meth. Cell Science,* 2000, vol. 22, 89-99
- **BANCHEREAU, M. ; R.M. STEINMAN.** *Nature,* 1998, vol. 329, 245-252
- **STEINMAN R.M. ; K. INABA.** *J. Leukoc. Biol.,* 1999, vol. 66, 205-208
- **WEIS W.I. ; TAYLOR M. E. ; DRICKAMER K.** *Immunol. Rev.,* 1998, vol. 163, 19-34
- **SANTIS A.G. et al.** *Eur. J. Immunol.,* 1994, vol. 24, 1692-7
- **HAMANN et al.** *Immunogenetics,* 1997, vol. 45, 295-300
- **YOKOYAMA W. M. et al.** *J. Immunol.,* 1991, vol. 147, 3229-3236
- **CHANG C et al.** *Eur. J. Immunol.,* 1995, vol. 25, 2433-2437
- **HOUCHINS J. P. et al.** *J. Exp. Med.,* 1991, vol. 173, 1017-1020
- **HOFER E. et al.** *Immunol. Today,* 1992, vol. 13, 429-430
- **BROWN M.G. et al.** *Genomics,* 1997, vol. 42, 16-25
- **SOBANOV Y. et al.** *Immunogenetics,* 1999, vol. 49, 99-105
- **LOPEZ-BOTET M. et al.** *Semin. Immunol.,* 2000, vol. 12, 109-119
- **HOGLUND P.** *Immunol. Rev.,* 1997, vol. 155, 11-28
- **ALCAMI A. ; U.H. KOSZINOWSKI.** *Immunol. Today,* 2000, vol. 21, 447-55
- **BAUER S. et al.** *Science,* 1999, vol. 285, 727-729
- **GLIENKE J. et al.** *Immunogenetics,* 1998, vol. 48, 163-173
- **DUCHLER M. et al.** *Eur. J. Immunol.,* 1995, vol. 25, 2923-31
- **BULL C. et al.** *Genes and Immunity,* 2000, vol. 1, 280-287
- **SAWAMURA T. et al.** *Nature,* 1997, vol. 386, 73-77
- **COLONNA M. ; SAMARIDIS J. ; ANGMAN L.** *Eur. J. Immunol.,* 2000, vol. 30, 697-704
- **HART D.N.** *Blood,* 1997, vol. 90, 3245-87
- **HOUCHINS J.P. et al.** *J. Exp. Med.,* 1991, vol. 173, 1017-1020
- **CHANG C. et al.** *Eur. J. Immunol.,* 1995, vol. 25, 2433-7
- **COLONNA M. et al.** *Eur. J. Immunol.,* 2000, vol. 30, 697-704
- **WEIS W. I. et al.** *Immunol. Rev.,* 1998, vol. 163, 19-34
- **ARIIZUMI et al.** *J. Biol. Chem.,* 2000, vol. 275, 20157-20167
- **SAWAMURA T.** *Nature,* 1997, vol. 386, 73-77
- **HOUCHINS J.P. et al.** *J. Exp. Med,* 1991, vol. 173, 1017-1020
- **ADAMS M.D. et al.** *Science,* 1991, vol. 252, 1651-1656
- **ADAMS M.D. et al.** *Nature,* 1992, vol. 355, 632-634
- **ADAMS M.D. et al.** *Nature,* 1995, vol. 377, 174
- **GENTZ et al.** *Proc. Natl Acad. Sci. USA,* 1989, vol. 86, 821-824
- **DAVIS et al.** BASIC METHODS IN MOLECULAR BIOLOGY. 1986
- **SAMBROOK et al.** MOLECULAR CLONING: A LABORATORY MANUAL. Cold Spring Harbor Laboratory Press, 1989
- **MITSUI et al.** *BBRC,* 1999, vol. 266, 115-122
- **CRUZ et al.** *FEBS letters,* 2001, vol. 488, 74-80
- **SCHEFFNER et al.** *Cell,* 1993, vol. 75, 495-505
- **ZHU et al.** *Nature,* 1999, vol. 400, 687-693
- **LI et al.** *PNAS,* 2000, vol. 98, 1619-1624
- **BISCHOFF ; PONSTINGL.** *Nature,* 1991, vol. 354, 80-82

- **WEIS W.I. et al.** *Immunol. Rev.,* 1998, vol. 163, 19-34
- **SOBANOV, Y. et al.** *Immunogenetics,* 1999, vol. 49, 99-105
- **HOGLUND P. et al.** *Immunol. Rev.,* 1997, vol. 155, 11-28
- **LANIER L. L. et al.** *Nature,* 1998, vol. 391, 703-707
- **CARRETERO M. et al.** *Eur J Immunol.,* 1997, vol. 27, 563-567
- **MYERS et al.** *Science,* 1985, vol. 230, 1242
- **COTTON et al.** *Proc. Natl Acad. Sci. USA,* 1985, vol. 85, 4397-4401
- **M. CHEE et al.** *Science,* 1996, vol. 274, 610-613
- **KOHLER, G. ; C. MILSTEIN.** *Nature,* 1975, vol. 256, 495-497
- **KOZBOR et al.** *Immunology Today,* 1983, vol. 4, 72
- **COLE et al.** MONOCLONAL ANTIBODIES AND CANCER THERAPY. Alan R. Liss, Inc, 1985, 77-96
- **COLIGAN et al.** *Current Protocols in Immunology,* 1991, vol. 1 (2
- **O'CONNOR.** *J. Neurochem.,* 1991, vol. 56, 560
- Oligodeoxynucleotides as Antisense Inhibitors of Gene Expression. CRC Press, 1988
- **LEE et al.** *Nucleic Acids Res.,* 1979, vol. 6, 3073
- **COONEY et al.** *Science,* 1988, vol. 241, 456
- **DERVAN et al.** *Science,* 1991, vol. 251, 1360
- Human Molecular Genetics. BIOS Scientific Publishers Ltd, 1996
- **FROHMAN et al.** *Proc. Nat. Acad. Sci USA,* 1988, vol. 85, 8998-9002
- **LANIER L.L. et al.** *Immunity,* 1998, vol. 8, 693-701
- **PALMIERI G. et al.** *J. Immunol.,* 1999, vol. 162, 7181-8
- **ARIIZUMI K. et al.** *J. Biol. Chem.,* 2000, vol. 275, 20157-20167